# EUROPEAN PATENT APPLICATION

(11) **EP 3 804 605 A1**
(43) Date of publication of application: **14.04.2021**
(21) Application number: 19816059.0
(22) Date of filing: 20.05.2019
(51) Int. Cl.: A61B 1/045, A61B 1/00

(54) **IMAGE PROCESSING DEVICE, ENDOSCOPE SYSTEM, AND IMAGE PROCESSING METHOD**

(30) Priority: 04.06.2018 JP 2018107152
(71) Applicant: FUJIFILM Corporation, Tokyo 106-8620 (JP)
(72) Inventor: KAMON, Shumpei, Ashigarakami-gun, Kanagawa 258-8538 (JP)
(74) Representative: Jeffrey, Philip Michael
(86) International application number: PCT/JP2019/019842
(87) International publication number: WO 2019/235195

(57) **Abstract**

The present invention is directed to providing an image processing apparatus, an endoscope system, and an image processing method that are capable of acquiring images by using a plurality of types of observation light as necessary while suppressing an influence on observation performed by a user. In an image processing apparatus according to a first aspect of the present invention, a first image is continuously acquired and displayed as a moving image by using first observation light until a still image acquisition instruction is received. When the still image acquisition instruction is received, a first image and a second image are acquired as still images by using first observation light and second observation light. After the still images have been acquired, a first image is acquired and displayed as a moving image again by using first observation light. Thus, the user is able to acquire still images by using first observation light and second observation light as necessary (for example, in response to a user instruction, or at a timing when acquisition of a still image is necessary such as when a region of interest is detected) while continuing observation with the first image, and is able to observe and classify a photographic subject.

## Description

### BACKGROUND OF THE INVENTION

### 1. Field of the Invention

The present invention relates to an image processing apparatus, an endoscope system, and an image processing method, and specifically relates to an image processing apparatus, an endoscope system, and an image processing method that acquire images by using a plurality of types of observation light.

### 2. Description of the Related Art

In medical practice, an image of a subject captured by using medical equipment is used in diagnosis, treatment, or the like. "What structure of a photographic subject is clearly (or unclearly) seen in a captured image" depends on the observation light used for imaging. For example, an image captured under special light, such as narrow-band light with a strong short-wavelength component, depicts blood vessels in a surface layer with a favorable contrast and is thus suitable for detecting a lesion. On the other hand, an image captured under special light with a strong long-wavelength component depicts blood vessels in a deep layer with a favorable contrast. Meanwhile, observation by a medical doctor is often performed by using normal light (white light), not special light. In this way, in imaging it is preferable to radiate observation light suitable for the usage purpose of an image or a target.

As a technique for switching observation light in this manner, for example, JP2017-153978A is known. JP2017-153978A describes an endoscope system that has a normal observation mode in which second white light is radiated to display a normal-light image and a special observation mode in which an oxygen saturation image is generated from an image obtained by alternately radiating first white light and second white light and the oxygen saturation image is displayed.

### SUMMARY OF THE INVENTION

In the case of switching between narrow-band light having a strong short wavelength component and normal light, an image generated by using special light (including an image generated by radiating a plurality of types of narrow-band light and an image generated by radiating narrow-band light having a short wavelength) may be unsuitable for observation to a user who is used to perform observation with a normal-light image, and a method for constantly acquiring an image by using special light during diagnosis may disturb the user in observation.

The present invention has been made in view of these circumstances, and an object of the present invention is to provide an image processing apparatus, an endoscope system, and an image processing method that are capable of acquiring images by using a plurality of types of observation light as necessary while suppressing an influence on observation performed by a user.

To achieve the above-described object, an image processing apparatus according to a first aspect of the present invention includes: an image acquiring unit that acquires a first image captured by using first observation light and a second image captured by using second observation light different from the first observation light; an acquisition instruction receiving unit that receives an acquisition instruction to acquire a still image; an image acquisition control unit that controls acquisition of the first image and the second image by the image acquiring unit; a display control unit that causes a display apparatus to display at least the first image; and a classifying unit that performs classification of at least a photographic subject that is seen in the second image. The image acquisition control unit causes the image acquiring unit to perform moving image acquisition processing of continuously acquiring the first image as a moving image until the acquisition instruction receiving unit receives the acquisition instruction, causes the image acquiring unit to perform still image acquisition processing of acquiring the first image and the second image as still images in response to receipt of the acquisition instruction, and causes the image acquiring unit to perform the moving image acquisition processing after the still image acquisition processing has finished.

In the case of acquiring images by using a plurality of types of observation light, it is preferable to acquire images by using a plurality of types of observation light as necessary while not disturbing observation by a user. However, in the above-mentioned JP2017-153978A, in the special observation mode, first white light and second white light are alternately radiated and an oxygen saturation image is displayed, and thus observation with normal light is disturbed. In contrast to the related art, in the first aspect, the first image is continuously acquired and displayed as a moving image by using the first observation light until a still image acquisition instruction is received. When a still image acquisition instruction is received, the first image and the second image are acquired as still images by using the first observation light and the second observation light, and then the first image is acquired and displayed again as a moving image by using the first observation light after the still images have been acquired. Accordingly, the user is able to acquire still images by using the first observation light and the second observation light as necessary (at a timing when acquisition of still images is necessary, for example, when a user instruction is provided or when a region of interest is detected) while continuing observation with the first image, and is able to perform classification of a photographic subject (a region of interest or the like) together with observation.

In the first aspect, one frame of a moving image can be acquired as a still image. In the case of imaging the inside of a living body, determination of the type of polyp (neoplastic or non-neoplastic), diagnosis of the stage of cancer, or the position in a lumen (an imaging position) can be performed as "classification". In the first aspect, the first image is continuously displayed. The second image can be displayed as necessary (for example, in response to input of a user instruction or in accordance with a result of processing the second image).

In the first aspect and the following individual aspects, one of the first observation light and the second observation light may be white light and the other may be narrow-band light, or both may be narrow-band light of different types. Each of the first observation light and the second observation light may be light emitted by a light source, or may be light generated by applying, to light emitted by a light source (for example, white light), a filter that allows a specific wavelength range to pass therethrough. In the case of using narrow-band light as the first observation light and/or the second observation light, the narrow-band light to be used may be narrow-band light radiated by a light source for narrow-band light, or may be narrow-band light generated by applying, to white light, a filter that allows a specific wavelength range to pass therethrough. In this case, the filter may be sequentially switched to radiate different types of narrow-band light at different timings.

In the first aspect, the first image captured by using the first observation light and the second image captured by using the second observation light are acquired. Because the second observation light is not used to capture the first image and the first observation light is not used to capture the second image, degradation of the image quality of the first image and the second image caused by insufficient wavelength separation does not occur.

In the first aspect and the following individual aspects, "the first observation light is different from the second observation light" means that at least one of the wavelength range or the spectrum is not identical between the first observation light and the second observation light. The first image and the second image may be medical images obtained by imaging a subject, such as a living body.

In the first aspect and the following individual aspects,. As a light source used to capture a medical image, a light source that generates light in a white range, light including a plurality of wavelengths (narrow-band light) as the white range, infrared light, or excitation light can be used. The medical image acquired in the first aspect may be a normal-light image acquired by radiating light in the white range or light in a plurality of wavelength ranges as the light in the white range, or may be a special-light image acquired on the basis of a normal-light image and having information of a specific wavelength range.

In this way, according to the first aspect, it is possible to acquire images by using a plurality of types of observation light in accordance with a purpose (observation, classification of a photographic subject, or the like) while suppressing an influence on observation performed by a user.

In an image processing apparatus according to a second aspect, in the first aspect, the image processing apparatus further includes a region-of-interest detecting unit that detects a region of interest from the first image acquired as the moving image. In a case where the region of interest has been detected, the image acquisition control unit instructs the acquisition instruction receiving unit to acquire the first image and the second image as the still images. In the second aspect, a still image can be automatically acquired (without an instruction from a user) in accordance with detection of a region of interest. The region-of-interest detecting unit is capable of performing region-of-interest detection processing on the first image that constitutes one frame of a moving image. The region of interest is also referred to as a region of concern.

In an image processing apparatus according to a third aspect, in the second aspect, the region-of-interest detecting unit detects the region of interest as the photographic subject from the first image and/or the second image as the still image, and the display control unit causes the display apparatus to display the first image and/or the second image as the still image such that the detected region of interest is emphasized. According to the third aspect, because the first image and/or the second image as the still image is displayed such that the region of interest is emphasized, the user is able to easily determine the position of the region of interest for which the first image and/or the second image has been acquired, and the region for which classification has been performed. If the region of interest is wrongly detected, the target for which the first image and/or the second image has been acquired can be determined, and thus the wrong detection can be easily determined. In the third aspect, the region of interest can be emphasized through marking with a specific figure, such as a rectangle, a circle, a cross, or an arrow, superimposition processing, change of color tone or gradation, frequency processing, or the like, but the emphasizing is not limited to these examples.

In an image processing apparatus according to a fourth aspect, in any one of the first to third aspects, the image processing apparatus further includes a classification result storing unit that stores a result of the classification in association with the first image and/or the second image. According to the fourth aspect, the relationship between the classification result and the first and second images becomes clear. In the fourth aspect, the classification result can be associated with the first image as a moving image, or the first image and/or the second image as a still image.

In an image processing apparatus according to a fifth aspect, in any one of the first to fourth aspects, the display control unit causes the display apparatus to display information indicating a result of the classification. In the fifth aspect, the information can be displayed by using, for example, characters, numerals, figures, symbols, colors, or the like corresponding to the classification result, and accordingly a user is able to easily recognize the classification result. The information may be displayed by being superimposed on an image, or may be displayed separately from the image.

In an image processing apparatus according to a sixth aspect, in the first or second aspect, the display control unit causes the display apparatus to display the first image and/or the second image as the still image. According to the sixth aspect, the user is able to check the still image (the first image and/or the second image) while performing observation with the first image (the moving image) and is accordingly able to determine to perform imaging again if the captured still image has a fault.

In an image processing apparatus according to a seventh aspect, in any one of the first to third aspects, the image processing apparatus further includes an image editing unit that performs image processing on the first image and/or the second image as the still image. The display control unit causes the display apparatus to display an image acquired through the image processing. In the seventh aspect, for example, image processing, such as color balance adjustment, blood vessel emphasis, feature quantity emphasis, difference emphasis, or combining of images that have undergone these processes, can be performed to generate an observation image, a classification (discrimination) image, and the like, and these images can be displayed.

In an image processing apparatus according to an eighth aspect, in any one of the first to seventh aspects, the image processing apparatus further includes: a parameter calculating unit that calculates a parameter for aligning the first image and the second image; and an image generating unit that generates an alignment first image by applying the parameter to the first image. The display control unit causes the display apparatus to display the alignment first image at a timing when the second image is acquired. In the case of acquiring an image by radiating only one of the first observation light and the second observation light, the first image is not acquired at a timing when the second image is acquired. However, in the eighth aspect, a parameter for alignment is applied to the first image to generate an alignment first image, and thus a substantial decrease in the frame rate of the first image can be prevented. In addition, change in the tint and structure of a photographic subject can be reduced between frames (between a frame of the first image and a frame of the alignment first image). In the eighth aspect and the following individual aspects, the "alignment first image" means "a first image at an imaging time of a second image, generated by applying an alignment parameter to a first image".

In the eighth aspect, the parameter calculating unit may calculate, as a parameter, a parameter about at least one of relative movement, rotation, or deformation between the first image and the second image. "Deformation" may include enlargement or reduction. In addition, the parameter calculating unit may calculate, as a parameter, a parameter for performing projective transformation between the first image and the second image, and the image generating unit may generate an alignment first image by performing projective transformation based on the calculated parameter on the first image.

In an image processing apparatus according to a ninth aspect, in the eighth aspect, the parameter calculating unit calculates the parameter for aligning the second image and the first image, the first image being captured at an imaging time that has a temporal difference smaller than or equal to a first threshold value from an imaging time of the second image. In a case where the temporal difference between the imaging times exceeds the first threshold value, an imaging range, an imaging angle, or the like may change because of a motion of a photographic subject or the like and the alignment accuracy may decrease. Thus, in the ninth aspect, the first image captured at an imaging time having a temporal difference smaller than or equal to the first threshold value from the imaging time of the second image is acquired. Accordingly, it is possible to generate an alignment first image with a small change in the structure of a photographic subject compared to the first image. In the ninth aspect, the first threshold value can be set in consideration of a condition, such as alignment accuracy.

In an image processing apparatus according to a tenth aspect, in the eighth aspect, the parameter calculating unit extracts a common wavelength component in an image signal of the first image and an image signal of the second image, the common wavelength component being common to a wavelength of the first observation light and a wavelength of the second observation light, performs at least any one of processing of weighting an image signal component of the first image of the common wavelength component to generate an image signal in which the image signal component of the first image of the common wavelength component is stronger than an image signal component of the first image of a component other than the common wavelength component, or processing of weighting an image signal component of the second image of the common wavelength component to generate an image signal in which the image signal component of the second image of the common wavelength component is stronger than an image signal component of the second image of a component other than the common wavelength component, and calculates a parameter for aligning the first image and the second image. Accordingly, it is possible to increase the alignment accuracy and acquire an image with a small change in the tint and structure of a photographic subject between frames (an alignment first image).

In an image processing apparatus according to an eleventh aspect, in the eighth aspect, the parameter calculating unit extracts a common wavelength component in an image signal of the first image and an image signal of the second image, the common wavelength component being common to a wavelength of the first observation light and a wavelength of the second observation light, generates an image signal component of the first image of the common wavelength component and an image signal component of the second image of the common wavelength component, and calculates a parameter for aligning the first image and the second image. Accordingly, it is possible to increase the alignment accuracy and acquire an image with a small change in the tint and structure of a photographic subject between frames (an alignment first image).

In an image processing apparatus according to a twelfth aspect, in any one of the first to eleventh aspects, at a timing when the second image is acquired, the display control unit causes the display apparatus to display the first image captured at an imaging time that has a temporal difference smaller than or equal to a second threshold value from an imaging time of the second image. According to the twelfth aspect, at the timing when the second image is acquired, display of the first image is continued, and thus it is possible to suppress an influence on observation caused by interruption of display of the first image or display of an image with a different tint. In the twelfth aspect, the second threshold value can be set in consideration of an influence on an image caused by a difference in imaging time (the position, orientation, or the like of a photographic subject).

In an image processing apparatus according to a thirteenth aspect, in any one of the first to twelfth aspects, the image acquiring unit acquires, as the second image, an image captured by using the second observation light, the second observation light being light whose center wavelength is shorter than a center wavelength of the first observation light. The structure of a photographic subject seen in an image varies according to the wavelength of observation light, and thus it is preferable to use observation light having a short wavelength to capture and detect a minute structure of a lesion or the like. In the thirteenth aspect, detection of a minute structure, classification of a photographic subject, or the like can be accurately performed by using the second image while observation is continued by displaying the first image.

In an image processing apparatus according to a fourteenth aspect, in any one of the first to thirteenth aspects, the acquisition instruction receiving unit receives, as the acquisition instruction, an acquisition instruction to acquire a still image from a user. According to the fourteenth aspect, the user is able to cause an image to be acquired at a desired timing.

To achieve the above-described object, an endoscope system according to a fifteenth aspect of the present invention includes: the image processing apparatus according to any one of the first to fourteenth aspects; the display apparatus; an endoscope that has an insertion section and a handheld operation section, the insertion section being to be inserted into a subject and having a tip rigid part, a bending part connected to a base end side of the tip rigid part, and a soft part connected to a base end side of the bending part, the handheld operation section being connected to a base end side of the insertion section; a light source apparatus that irradiates the subject with the first observation light or the second observation light; and an imaging unit that has an imaging lens which forms an optical image of the subject and an imaging device on which the optical image is formed by the imaging lens. The imaging lens is provided at the tip rigid part. The endoscope system according to the fifteenth aspect includes the image processing apparatus according to any one of the first to fourteenth aspects, and is thus capable of acquiring an image by using a plurality of types of observation light as necessary while suppressing an influence on observation performed by a user.

The endoscope system according to the fifteenth aspect includes the image processing apparatus according to any one of the first to fourteenth aspects, and thus an advantageous effect of including the image processing apparatus is acquired. That is, because the second image is not acquired in a case where the necessity for the second image is low (for example, a case where a region of interest or the like is not detected and classification of a photographic subject is not necessary), and thus it is possible to prevent that increased repetition of radiation and non-radiation of observation light unnecessarily hastens degradation of the light source.

In the fifteenth aspect, light emitted by the light source may be used as observation light, or light generated by applying, to light emitted by the light source, a filter that allows a specific wavelength range to pass therethrough may be used as observation light. For example, in the case of using narrow-band light as the first observation light and/or the second observation light, light radiated by a narrow-band light source may be used as observation light, or light generated by applying, to white light, a filter that allows a specific wavelength range to pass therethrough may be used as observation light. In this case, the filter applied to white light may be sequentially switched to radiate different types of narrow-band light at different timings.

In an endoscope system according to a sixteenth aspect, in the fifteenth aspect, the light source apparatus irradiates the subject with the first observation light, the first observation light being white light including light in a red wavelength range, a blue wavelength range, and a green wavelength range, and irradiates the subject with the second observation light, the second observation light being narrow-band light corresponding to any one of the red wavelength range, the blue wavelength range, and the green wavelength range. According to the sixteenth aspect, it is possible to perform detection and classification of a region of interest by using the second image captured by using narrow-band light (second observation light) while performing observation by displaying the first image captured by using white light (first observation light). Alternatively, narrow-band light corresponding to a purple wavelength range and an infrared wavelength range may be used.

In an endoscope system according to a seventeenth aspect, in the sixteenth aspect, the light source apparatus includes a white-light laser light source that radiates white-light laser as excitation light; a fluorescent body that emits the white light as the first observation light when irradiated with the white-light laser; and a narrow-band-light laser light source that radiates the narrow-band light as the second observation light. In the case of using a laser light source for excitation light to acquire white light as the first observation light, a high second image acquisition frequency increases repetition of radiation and non-radiation of the first observation light. Accordingly, repetition of excitation and non-excitation of the white-light laser light source increases and degradation of the light source may be hastened. However, the endoscope system according to the seventeenth aspect includes the image processing apparatus according to any one of the first to fourteenth aspects, and thus an advantageous effect of including the image processing apparatus is acquired. That is, because the second image is not acquired in a case where the necessity for the second image is low (for example, a case where a region of interest or the like is not detected and classification is not necessary), and thus it is possible to prevent that increased repetition of radiation and non-radiation of observation light unnecessarily hastens degradation of the light source.

In an endoscope system according to an eighteenth aspect, in the sixteenth aspect, the light source apparatus includes a white light source that emits the white light; a white-light filter that allows the white light to pass therethrough; a narrow-band-light filter that allows a component of the narrow-band light in the white light to pass therethrough; and a first filter switching control unit that inserts the white-light filter or the narrow-band-light filter to an optical path of the white light emitted by the white light source. In the case of generating a plurality of types of observation light (white light and narrow-band light) by switching a filter, lack of synchronization between the switching of the filter and the read-out timing of an image sensor (an imaging device) may cause an imbalance in the color of the first image and/or the second image. However, since the endoscope system according to the eighteenth aspect includes the image processing apparatus according to any one of the first to fourteenth aspects, an advantageous effect of including the image processing apparatus is acquired. That is, it is possible to reduce the possibility that the second image is acquired and the number of times of switching of the light source or the filter increases even in a case where the necessity for the second image is low (for example, a case were a region of interest is not detected and classification is unnecessary), and the color balance of the first image and/or the second image is lost.

In an endoscope system according to a nineteenth aspect, in the fifteenth aspect, the light source apparatus irradiates the subject with the first observation light, the first observation light being first narrow-band light that corresponds to any one of a red wavelength range, a blue wavelength range, and a green wavelength range, and irradiates the subject with the second observation light, the second observation light being second narrow-band light that corresponds to any one of the red wavelength range, the blue wavelength range, and the green wavelength range and that has a wavelength range different from a wavelength range of the first narrow-band light. The nineteenth aspect defines an aspect of using a plurality of types of narrow-band light. For example, a combination of a plurality of types of blue narrow-band light having different wavelengths, a combination of blue narrow-band light and green narrow-band light, a combination of a plurality of types of red narrow-band light having different wavelengths, or the like may be used, but the observation light is not limited to these combinations. Narrow-band light corresponding to a purple wavelength range and an infrared wavelength range may be used.

In an endoscope system according to a twentieth aspect, in the nineteenth aspect, the light source apparatus includes a white light source that emits white light including light in the red wavelength range, the blue wavelength range, and the green wavelength range; a first-narrow-band-light filter that allows a component of the first narrow-band light in the white light to pass therethrough; a second-narrow-band-light filter that allows a component of the second narrow-band light in the white light to pass therethrough; and a second filter switching control unit that inserts the first-narrow-band-light filter or the second-narrow-band-light filter to an optical path of the white light emitted by the white light source. In the case of generating a plurality of types of observation light (first narrow-band light and second narrow-band light) by switching a filter by the second filter switching control unit, lack of synchronization between the switching of the filter and the read-out timing of an image sensor (an imaging device) may cause an imbalance in the color of the first image and/or the second image. However, since the endoscope system according to the twentieth aspect includes the image processing apparatus according to any one of the first to fourteenth aspects, an advantageous effect of including the image processing apparatus is acquired. That is, it is possible to reduce the possibility that the second image is acquired and the number of times of switching of the light source or the filter increases even in a case where the necessity for the second image is low (for example, a case were a region of interest is not detected and classification is unnecessary), and the color balance of the first image and/or the second image is lost.

To achieve the above-described object, an image processing method according to a twenty-first aspect of the present invention is an image processing method for an image processing apparatus including an image acquiring unit that acquires a first image captured by using first observation light and a second image captured by using second observation light different from the first observation light. The image processing method includes: an acquisition instruction reception step of receiving an acquisition instruction to acquire a still image; an image acquisition control step of controlling acquisition of the first image and the second image by the image acquiring unit; a display control step of causing a display apparatus to display at least the first image; and a classification step of performing classification of at least a photographic subject that is seen in the second image. The image acquisition control step causes the image acquiring unit to perform moving image acquisition processing of continuously acquiring the first image as a moving image until the acquisition instruction is received in the acquisition instruction reception step, causes the image acquiring unit to perform still image acquisition processing of acquiring the first image and the second image as still images in response to receipt of the acquisition instruction, and causes the image acquiring unit to perform the moving image acquisition processing after the still image acquisition processing has finished. According to the twenty-first aspect, as in the first aspect, images can be acquired by using a plurality of types of observation light as necessary, with an influence on observation performed by a user being suppressed.

The image processing method according to the twenty-first aspect may further include configurations similar to those according to the second to fourteenth aspects. In addition, a program that causes the image processing apparatus or the endoscope system to execute the image processing methods according to these aspects, and a non-transitory recording medium storing a computer-readable code of the program may be included in an aspect of the present invention.

As described above, the image processing apparatus, the endoscope system, and the image processing method according to the present invention are capable of acquiring images by using a plurality of types of observation light as necessary while suppressing an influence on observation performed by a user.

### BRIEF DESCRIPTION OF THE DRAWINGS

Fig. 1 is an external appearance diagram of an endoscope system according to a first embodiment;
Fig. 2 is a block diagram illustrating the configuration of the endoscope system;
Fig. 3 is a diagram illustrating the configuration of a tip rigid part of an endoscope;
Fig. 4 is a diagram illustrating a functional configuration of an image processing unit;

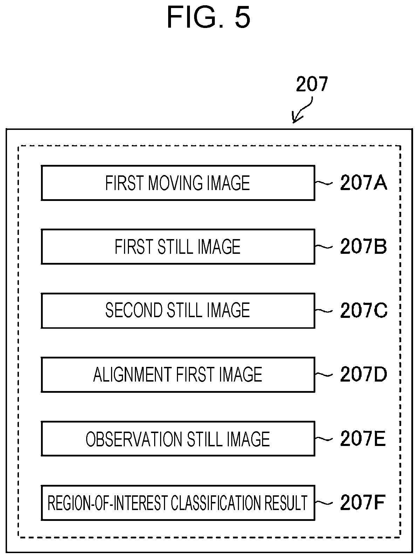

Fig. 5 is a diagram illustrating information recorded in a recording unit;
Fig. 6 is a flowchart illustrating a procedure of image processing;
Fig. 7 is a flowchart (continued from Fig. 6) illustrating the procedure of image processing;
Figs. 8A and 8B are diagrams illustrating a state in which a moving image and a still image are acquired;
Figs. 9A and 9B are diagrams illustrating examples of displaying a moving image and a still image;
Fig. 10 is a diagram illustrating an example of displaying a still image;
Fig. 11 is a diagram illustrating a state in which a discrimination result of a region of interest is displayed together with images;
Fig. 12 is a diagram illustrating an example of displaying a region of interest in an emphasized manner;
Figs. 13A and 13B are other diagrams each illustrating an example of displaying a region of interest in an emphasized manner;
Fig. 14 is a diagram illustrating a state in which images and classification results of regions of interest are stored in association with each other;
Fig. 15 is another diagram illustrating a state in which images and classification results of regions of interest are stored in association with each other;
Fig. 16 is a flowchart illustrating processing for an alignment first image;
Figs. 17A and 17B are diagrams illustrating a state of creating an alignment first image;
Fig. 18 is a diagram illustrating a state in which a blue light component is weighted in a first still image;
Fig. 19 is a diagram illustrating an example of an alignment first image;
Fig. 20 is a diagram illustrating an example of the configuration of a light source;
Fig. 21 is a diagram illustrating another example of the configuration of a light source;
Figs. 22A and 22B are diagrams illustrating examples of a rotary filter; and
Figs. 23A and 23B are diagrams illustrating other examples of a rotary filter.

### DESCRIPTION OF THE PREFERRED EMBODIMENTS

Hereinafter, an embodiment of an image processing apparatus, an endoscope system, and an image processing method according to the present invention will be described in detail with reference to the attached drawings. In the following description, a moving image acquired by radiating first observation light may be referred to as a "first moving image", and still images respectively acquired by radiating first observation light and second observation light may be referred to as a "first still image" and a "second still image", respectively.

### First Embodiment

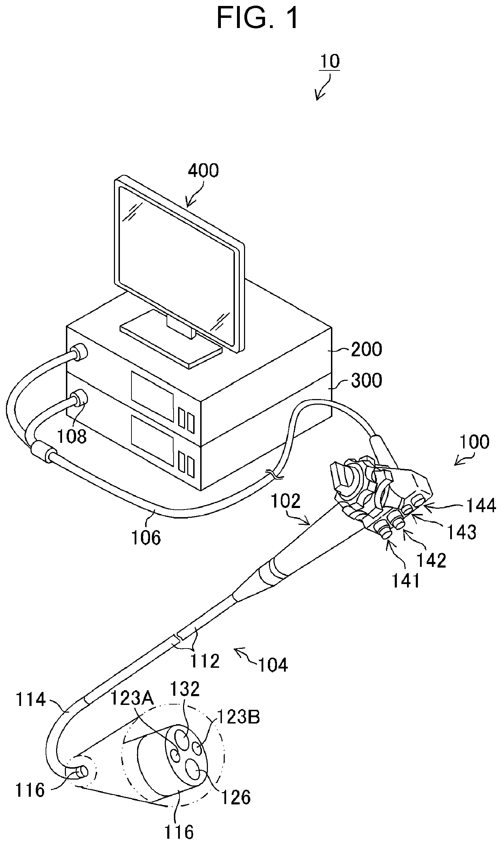

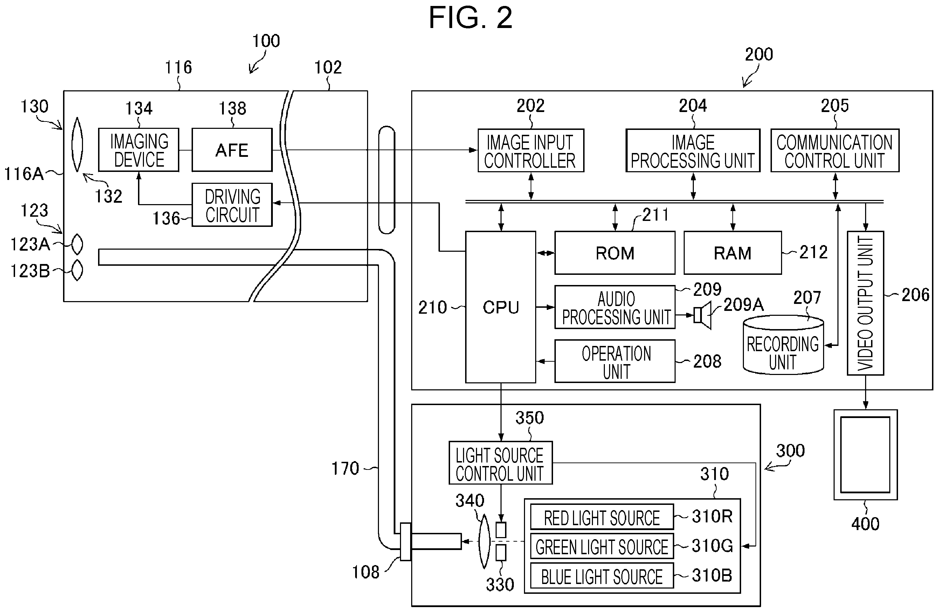

Fig. 1 is an external appearance diagram illustrating an endoscope system 10 (an image processing apparatus, a diagnosis assistance apparatus, an endoscope system, a medical image processing apparatus) according to a first embodiment, and Fig. 2 is a block diagram illustrating the configuration of a main part of the endoscope system 10. As illustrated in Figs. 1 and 2, the endoscope system 10 is constituted by an endoscope main body 100 (an endoscope), a processor 200 (a processor, an image processing apparatus, a medical image processing apparatus), a light source apparatus 300 (a light source apparatus), and a monitor 400 (a display apparatus).

### Configuration of Endoscope Main Body

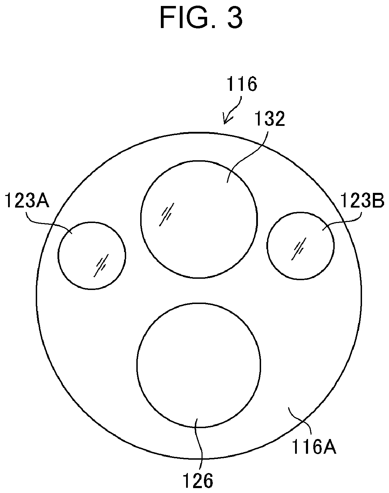

The endoscope main body 100 includes a handheld operation section 102 (a handheld operation section) and an insertion section 104 (an insertion section) that communicates with the handheld operation section 102. An operator (a user) operates the handheld operation section 102 while grasping it and inserts the insertion section 104 into a body of a subject (a living body) to perform observation. The handheld operation section 102 is provided with an air/water supply button 141, a suction button 142, a function button 143 to which various functions are allocated, and an imaging button 144 for receiving an imaging instruction operation (a still image, a moving image). The insertion section 104 is constituted by a soft part 112 (a soft part), a bending part 114 (a bending part), and a tip rigid part 116 (a tip rigid part), which are arranged in this order from the handheld operation section 102 side. That is, the bending part 114 is connected to a base end side of the tip rigid part 116, and the soft part 112 is connected to a base end side of the bending part 114. The handheld operation section 102 is connected to a base end side of the insertion section 104. The user is able to change the orientation of the tip rigid part 116 in an up, down, left, or right direction by causing the bending part 114 to bend by operating the handheld operation section 102. The tip rigid part 116 is provided with an imaging optical system 130 (an imaging unit), an illumination unit 123, a forceps port 126, and so forth (see Fig. 1 to Fig. 3).

During observation or treatment, an operation of an operation unit 208 (see Fig. 2) enables white light and/or narrow-band light (one or more of red narrow-band light, green narrow-band light, and blue narrow-band light) to be radiated from illumination lenses 123A and 123B of the illumination unit 123. In addition, an operation of the air/water supply button 141 enables washing water to be ejected from a water supply nozzle that is not illustrated, so that an imaging lens 132 (an imaging lens, an imaging unit) of the imaging optical system 130 and the illumination lenses 123A and 123B can be washed. The forceps port 126 opening in the tip rigid part 116 communicates with a pipe line that is not illustrated, so that a treatment tool that is not illustrated and is for extirpating a tumor or the like can be inserted into the pipe line and necessary treatment can be given to a subject by moving the treatment tool forward or backward as appropriate.

As illustrated in Fig. 1 to Fig. 3, the imaging lens 132 (an imaging unit) is disposed on a distal-end-side surface 116A of the tip rigid part 116. An imaging device 134 (an imaging device, an imaging unit) of a complementary metal-oxide semiconductor (CMOS) type, a driving circuit 136, and an analog front end (AFE) 138 are disposed behind the imaging lens 132, and these elements output an image signal. The imaging device 134 is a color imaging device and includes a plurality of pixels constituted by a plurality of light-receiving elements arranged in a matrix (arranged two-dimensionally) in a specific pattern arrangement (Bayer arrangement, X-Trans (registered trademark) arrangement, honeycomb arrangement, or the like). Each pixel of the imaging device 134 includes a microlens, a red (R), green (G), or blue (B) color filter, and a photoelectric conversion unit (a photodiode or the like). The imaging optical system 130 is capable of generating a color image from pixel signals of three colors, red, green, and blue, and is also capable of generating an image from pixel signals of any one or two colors among red, green, and blue. In the first embodiment, a description will be given of a case where the imaging device 134 is a CMOS-type imaging device, but the imaging device 134 may be of a charge coupled device (CCD) type. Each pixel of the imaging device 134 may further include a purple color filter corresponding to a purple light source and/or an infrared filter corresponding to an infrared light source.

An optical image of a subject (a tumor portion, a lesion portion) is formed on a light-receiving surface (an imaging surface) of the imaging device 134 by the imaging lens 132, converted into an electric signal, output to the processor 200 through a signal cable that is not illustrated, and converted into a video signal. Accordingly, an observation image is displayed on the monitor 400, which is connected to the processor 200.

The illumination lenses 123A and 123B of the illumination unit 123 are provided next to the imaging lens 132 on the distal-end-side surface 116A of the tip rigid part 116. An emission end of a light guide 170, which will be described below, is disposed behind the illumination lenses 123A and 123B. The light guide 170 extends through the insertion section 104, the handheld operation section 102, and a universal cable 106, and an incidence end of the light guide 170 is located in a light guide connector 108.

### Configuration of Light Source Apparatus

As illustrated in Fig. 2, the light source apparatus 300 is constituted by a light source 310 for illumination, a diaphragm 330, a condenser lens 340, a light source control unit 350, and so forth, and causes observation light to enter the light guide 170. The light source 310 includes a red light source 310R, a green light source 310G, and a blue light source 310B that emit red narrow-band light, green narrow-band light, and blue narrow-band light, respectively, and is capable of radiating red narrow-band light, green narrow-band light, and blue narrow-band light. The illuminance of observation light from the light source 310 is controlled by the light source control unit 350, which is capable of decreasing the illuminance of observation light or stopping illumination as necessary.

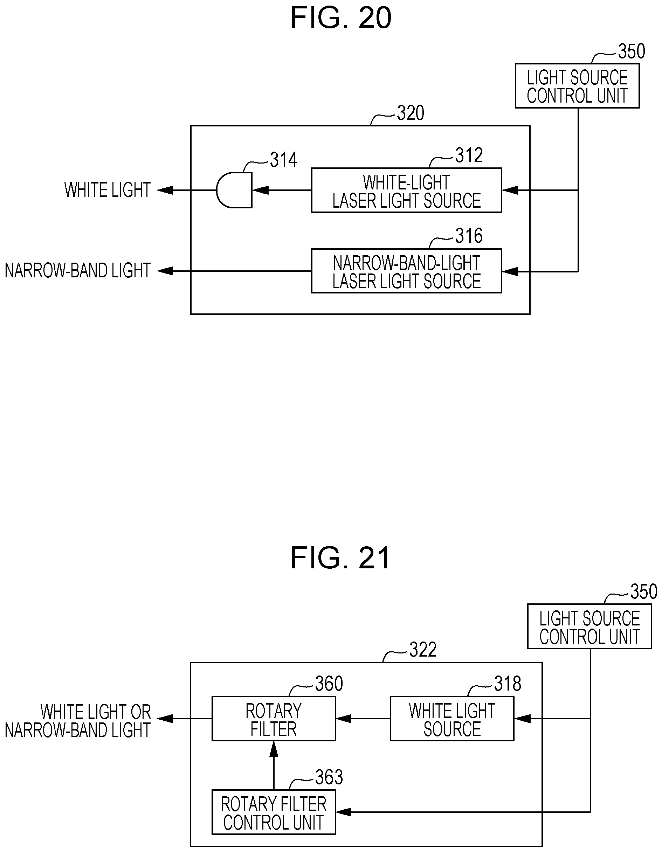

The light source 310 is capable of emitting red narrow-band light, green narrow-band light, and blue narrow-band light in any combination. For example, the light source 310 is capable of simultaneously emitting red narrow-band light, green narrow-band light, and blue narrow-band light to radiate white light (normal light) as observation light, and is also capable of emitting any one or two of red narrow-band light, green narrow-band light, and blue narrow-band light to radiate narrow-band light (special light). The light source 310 may further include a purple light source that radiates purple light (an example of narrow-band light) and/or an infrared light source that radiates infrared light (an example of narrow-band light). Alternatively, with use of a light source that radiates white light and a filter that allows white light and each narrow-band light to pass therethrough, white light or narrow-band light may be radiated as observation light (see, for example, Figs. 20 to 23B).

### Wavelength Range of Light Source

The light source 310 may be a light source that generates light in a white range or light in a plurality of wavelength ranges as the light in the white range, or may be a light source that generates light in a specific wavelength range narrower than the white wavelength range. The specific wavelength range may be a blue range or green range in a visible range, or may be a red range in the visible range. In a case where the specific wavelength range is the blue range or green range in the visible range, the specific wavelength range may include a wavelength range of 390 nm or more and 450 nm or less or a wavelength range of 530 nm or more and 550 nm or less, and the light in the specific wavelength range may have a peak wavelength in the wavelength range of 390 nm or more and 450 nm or less or the wavelength range of 530 nm or more and 550 nm or less. In a case where the specific wavelength range is the red range in the visible range, the specific wavelength range may include a wavelength range of 585 nm or more and 615 nm or less or a wavelength range of 610 nm or more and 730 nm or less, and the light in the specific wavelength range may have a peak wavelength in the wavelength range of 585 nm or more and 615 nm or less or the wavelength range of 610 nm or more and 730 nm or less.

The above-described specific wavelength range may include a wavelength range in which a light absorption coefficient is different between oxyhemoglobin and deoxyhemoglobin, and the light in the specific wavelength range may have a peak wavelength in the wavelength range in which the light absorption coefficient is different between oxyhemoglobin and deoxyhemoglobin. In this case, the specific wavelength range may include a wavelength range of 400 ± 10 nm, a wavelength range of 440 ± 10 nm, a wavelength range of 470 ± 10 nm, or a wavelength range of 600 nm or more and 750 nm or less, and the light in the specific wavelength range may have a peak wavelength in the wavelength range of 400 ± 10 nm, the wavelength range of 440 ± 10 nm, the wavelength range of 470 ± 10 nm, or the wavelength range of 600 nm or more and 750 nm or less.

The wavelength range of the light generated by the light source 310 may include a wavelength range of 790 nm or more and 820 nm or less or a wavelength range of 905 nm or more and 970 nm or less, and the light generated by the light source 310 may have a peak wavelength in the wavelength range of 790 nm or more and 820 nm or less or the wavelength range of 905 nm or more and 970 nm or less.

Alternatively, the light source 310 may include a light source that radiates excitation light whose peak is 390 nm or more and 470 nm or less. In this case, a medical image (an inside-of-living-body image) having information about fluorescence emitted by a fluorescent substance in a subject (a living body) can be acquired. In the case of acquiring a fluorescence image, a pigment for a fluorescence method (fluorescein, acridine orange, or the like) may be used.

It is preferable that the type of the light source 310 (a laser light source, a xenon light source, a light-emitting diode (LED) light source, or the like), the wavelength of the light source 310, the presence or absence of a filter for the light source 310, and so forth be determined in accordance with the type of photographic subject, the purpose of observation, or the like. It is also preferable that, during observation, the wavelengths of observation light be combined and/or switched in accordance with the type of photographic subject, the purpose of observation, or the like. In the case of switching the wavelength, for example, a disc-shaped filter (a rotary color filter) that is disposed in front of the light source and that is provided with a filter for transmitting or blocking light of a specific wavelength may be rotated to switch the wavelength of light to be radiated (see Figs. 20 to 23B).

The imaging device used to carry out the present invention is not limited to a color imaging device in which color filters are disposed for the individual pixels, such as the imaging device 134, and may be a monochrome imaging device. In the case of using a monochrome imaging device, imaging can be performed in a frame sequential (color sequential) manner by sequentially switching the wavelength of observation light. For example, the wavelength of outgoing observation light may be sequentially switched among blue, green, and red, or wide-band light (white light) may be radiated and the wavelength of outgoing observation light may be switched by using a rotary color filter (red, green, blue, and the like). Alternatively, one or a plurality of types of narrow-band light (green, blue, and the like) may be radiated and the wavelength of outgoing observation light may be switched by using a rotary color filter (green, blue, and the like). The narrow-band light may be infrared light of two or more different wavelengths (first narrow-band light and second narrow-band light).

As a result of connecting the light guide connector 108 (see Fig. 1) to the light source apparatus 300, observation light radiated by the light source apparatus 300 is transmitted through the light guide 170 to the illumination lenses 123A and 123B and is radiated from the illumination lenses 123A and 123B to an observation range.

### Configuration of Processor

The configuration of the processor 200 will be described with reference to Fig. 2. In the processor 200, an image input controller 202 receives an image signal output from the endoscope main body 100, an image processing unit 204 performs necessary image processing thereon, and a video output unit 206 outputs a resulting image signal. Accordingly, an observation image (an inside-of-living-body image) is displayed on the monitor 400 (a display apparatus). These processing operations are performed under control by a central processing unit (CPU) 210. Specifically, the CPU 210 has functions as an image acquiring unit, an acquisition instruction receiving unit, an image acquisition control unit, a display control unit, a classifying unit, a region-of-interest detecting unit, a classification result storing unit, an image editing unit, a parameter calculating unit, and an image generating unit. A communication control unit 205 controls communication with a hospital information system (HIS), a hospital local area network (LAN), and the like that are not illustrated. In a recording unit 207, an image of a photographic subject (a medical image, a captured image), information indicating a result of detection and/or classification of a region of interest, and the like are recorded. An audio processing unit 209 outputs a message (sound) or the like based on the result of detection and/or classification of the region of interest from a speaker 209A under control by the CPU 210 and the image processing unit 204.

A read only memory (ROM) 211 is a nonvolatile storage element (a non-transitory recording medium) and stores a computer-readable code of a program that causes the CPU 210 and/or the image processing unit 204 (an image processing apparatus, a computer) to execute the image processing method according to the present invention. A random access memory (RAM) 212 is a storage element for temporary storage in various processing operations and can be used as a buffer when acquiring an image.

### Functions of Image Processing Unit

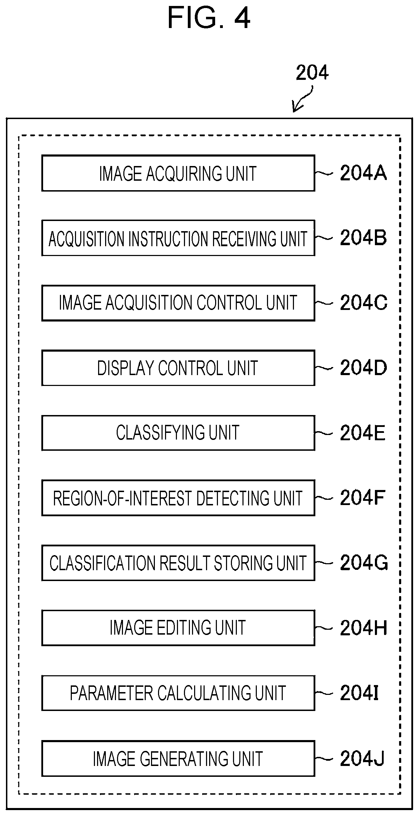

Fig. 4 is a diagram illustrating a functional configuration of the image processing unit 204 (a medical image acquiring unit, a medical image analysis processing unit, a medical image analysis result acquiring unit). The image processing unit 204 has an image acquiring unit 204A (an image acquiring unit), an acquisition instruction receiving unit 204B (an acquisition instruction receiving unit), an image acquisition control unit 204C (an image acquisition control unit), a display control unit 204D (a display control unit), a classifying unit 204E (a classifying unit), a region-of-interest detecting unit 204F (a region-of-interest detecting unit), a classification result storing unit 204G (a classification result storing unit), an image editing unit 204H (an image editing unit), a parameter calculating unit 2041 (a parameter calculating unit), and an image generating unit 204J (an image generating unit). The classifying unit 204E and the region-of-interest detecting unit 204F also operate as a medical image analysis processing unit.

In addition, the image processing unit 204 may include a special-light image acquiring unit that acquires a special-light image having information about a specific wavelength range on the basis of a normal-light image that is acquired by radiating light in the white range or light in a plurality of wavelength ranges as the light in the white range. In this case, a signal in the specific wavelength range can be acquired through computation based on color information of RGB (R: red, G: green, B: blue) or CMY (C: cyan, M: magenta, Y: yellow) included in the normal-light image.

In addition, the image processing unit 204 may include a feature quantity image generating unit that generates a feature quantity image through computation based on at least one of a normal-light image that is acquired by radiating light in the white range or light in a plurality of wavelength ranges as the light in the white range or a special-light image that is acquired by radiating light in a specific wavelength range, and may acquire and display the feature quantity image as a medical image. The image editing unit 204H may have a function of the feature quantity image generating unit.

The processing operations using these functions of the image processing unit 204 will be described in detail below. The processing operations using these functions are performed under control by the CPU 210.

The above-described functions of the image processing unit 204 can be implemented by using various types of processors. The various types of processors include, for example, a central processing unit (CPU) which is a general-purpose processor that executes software (program) to implement various functions. Also, the various types of processors include a graphics processing unit (GPU) which is a processor dedicated to image processing, and a programmable logic device (PLD) which is a processor whose circuit configuration is changeable after manufacturing, such as a field programmable gate array (FPGA). Furthermore, the various types of processors include a dedicated electric circuit which is a processor having a circuit configuration designed exclusively for executing specific processing, such as an application specific integrated circuit (ASIC).

The function of each unit may be implemented by one processor or may be implemented by a plurality of processors of the same type or different types (for example, a combination of a plurality of FPGAs, a combination of a CPU and an FPGA, or a combination of a CPU and a GPU). A plurality of functions may be implemented by one processor. A first example of implementing a plurality of functions by one processor is that a combination of one or more CPUs and software constitute one processor and the one processor implements the plurality of functions, as represented by a computer, such as a main body of an image processing apparatus or a server. A second example is that a processor that implements the functions of an entire system by one integrated circuit (IC) chip is used, as represented by a system on chip (SoC). In this way, various functions are configured as a hardware structure by using one or more of the above-described various types of processors. Furthermore, the hardware structure of the various types of processors is, more specifically, electric circuitry formed by combining circuit elements such as semiconductor elements.

When the above-described processor or electric circuitry executes the software (program), a processor (computer)-readable code of the software to be executed is stored in a non-transitory recording medium, such as a read only memory (ROM), and the processor refers to the software. The software stored in the non-transitory recording medium includes a program for executing input of an image and measurement of a photographic subject. The code may be recorded on a non-transitory recording medium, such as a magneto-optical recording device of various types or a semiconductor memory, instead of the ROM. In the processing using the software, a random access memory (RAM) may be used as a transitory storage region, for example, and data stored in an electrically erasable and programmable read only memory (EEPROM) that is not illustrated can be referred to, for example.

### Configuration of Operation Unit

The processor 200 includes the operation unit 208. The operation unit 208 includes an operation mode setting switch or the like that is not illustrated and is capable of setting the wavelength of observation light (white light or narrow-band light, which narrow-band light is to be used in the case of narrow-band light). In addition, the operation unit 208 includes a keyboard and a mouse that are not illustrated. A user is able to perform operations of setting an imaging condition and a display condition via these devices or provide an instruction to capture (acquire) a moving image or a still image (an instruction to capture a moving image or a still image may be provided by using the imaging button 144). These setting operations may be performed via a foot switch that is not illustrated, or may be performed by using a voice, a line of sight, a gesture, or the like.

### Configuration of Recording Unit

The recording unit 207 (a recording device) is configured including a non-transitory recording medium, such as a magneto-optical recording medium of various types or a semiconductor memory, and a control unit for the recording medium, and stores a first moving image 207A (a first image), a first still image 207B (a first image), a second still image 207C (a second image), an alignment first image 207D, an observation still image 207E, a region-of-interest classification result 207F, and the like in association with each other. These images and information are displayed on the monitor 400 as a result of an operation performed via the operation unit 208 and control by the CPU 210 and/or the image processing unit 204.

In addition to the above-described images, an analysis result about either or both of a region of interest (a region of concern), which is a region to be focused on included in a medical image, and the presence or absence of a target to be focused on may be recorded in the recording unit 207 (a recording device). In this case, the image processing unit 204 (a medical image analysis processing unit, a medical image analysis result acquiring unit) is capable of acquiring the analysis result from the recording unit 207 and displaying the analysis result on the monitor 400.

### Configuration of Display Apparatus

The monitor 400 (a display apparatus) displays the first moving image 207A (a first image), the first still image 207B (a first image), the second still image 207C (a second image), the alignment first image 207D, the observation still image 207E, the region-of-interest classification result 207F, and the like as a result of an operation performed via the operation unit 208 and control by the CPU 210 and/or the image processing unit 204. The monitor 400 has a touch panel that is not illustrated and that is for performing an imaging condition setting operation and/ or a display condition setting operation.

### Image Processing Method

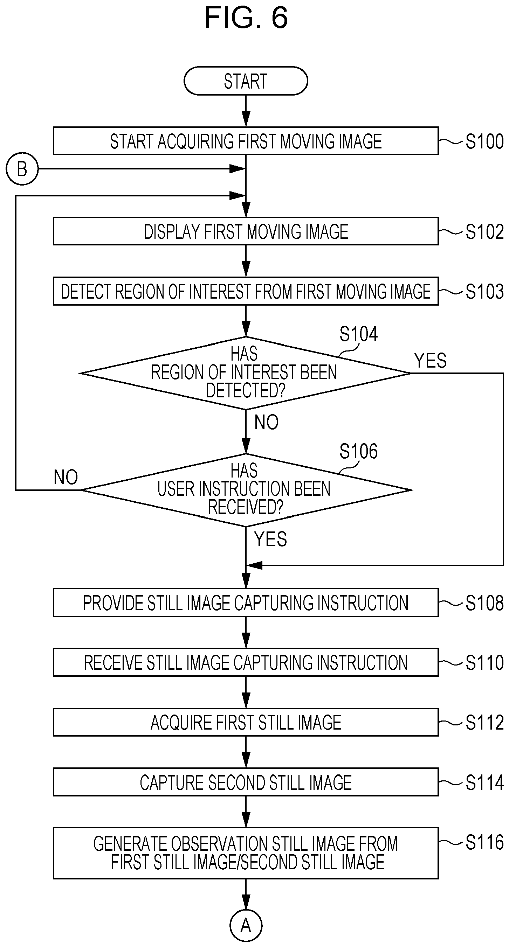

An image processing method using the endoscope system 10 having the above-described configuration will be described. Figs. 6 to 7 are flowcharts illustrating the procedure of an image processing method according to the first embodiment.

### Observation Light of First Image and Second Image

In the first embodiment, a description will be given of a case where a white-light image (a normal-light image) using white light as observation light (first observation light) is acquired as a first image and a blue-light image (a special-light image) using blue light which is narrow-band light (the center wavelength is shorter than that of the first observation light) as observation light (second observation light) is acquired as a second image. However, in the present invention, the observation light is not limited to such a combination. For example, the second image may be a special-light image acquired by using green light, red light, infrared light, purple light, or the like which is narrow-band light as observation light. Alternatively, a first image and a second image may be acquired by using first observation light and second observation light each of which is narrow-band light (for example, first narrow-band light and second narrow-band light, such as blue light and green light or red light beams having different wavelengths). In the first embodiment, it is assumed that a first image and a second image are captured by radiating only first observation light or only second observation light in one frame.

### Acquisition of First Moving Image

The image acquiring unit 204A controls the light source control unit 350 to cause the red light source 310R, the green light source 310G, and the blue light source 310B to emit light and irradiate a subject with white light (first observation light), and the imaging optical system 130, the imaging device 134, and so forth capture a first moving image (a first image, a normal-light image) of the subject (step S100: an image acquisition control step, moving image acquisition processing). Specifically, the image acquiring unit 204A sequentially acquires frame images constituting a moving image at a rate of 30 frames per second, 60 frames per second, or the like. The image acquiring unit 204A acquires (receives) the captured first moving image via the image input controller 202 (step S100: an image acquisition step). The display control unit 204D displays the acquired first moving image on the monitor 400 (a display apparatus) (step S102: a display control step).

### Detection of Region of Interest

The region-of-interest detecting unit 204F (a region-of-interest detecting unit) detects a region of interest from each frame of the acquired first moving image (step S103: a first region-of-interest detection step). Detection of a region of interest can be performed by the region-of-interest detecting unit 204F that includes, for example, a known computer aided diagnosis (CAD) system. Specifically, for example, a region of interest (a region of interest which is a region to be focused on) and the presence or absence of a target (a target to be focused on) in the region of interest can be extracted on the basis of a feature quantity of pixels of a medical image. In this case, the region-of-interest detecting unit 204F divides a detection target image into a plurality of rectangular regions, for example, and sets the individual rectangular regions obtained through division as local regions. The region-of-interest detecting unit 204F calculates, for each local region of the detection target image, a feature quantity (for example, a hue) of the pixels in the local region, and determines a local region having a specific hue among the local regions to be a region of interest. In step S103, "detects a region of interest" means "performs detection processing on an image".

### Detection of Region of Interest Based on Deep Learning Algorithm

Detection of a region of interest may be performed by using a result of deep learning. For example, every time a new image is recorded in the recording unit 207 (or every time a new image is captured), the region-of-interest detecting unit 204F performs image analysis processing using deep learning on the basis of a deep learning algorithm, thereby analyzing whether or not the image includes a region of interest. The deep learning algorithm is an algorithm of recognizing whether or not the image includes a region of interest by using a known method of a convolutional neural network, that is, repetition of a convolutional layer and a pooling layer, a fully connected layer, and an output layer. The image analysis processing using deep learning may use a learner generated by giving images labeled with "is a region of interest" or "is not a region of interest" as training data. "Whether or not to perform such machine learning" and/or "whether or not to use a learning result" may be set in accordance with a user operation via the operation unit 208 and the monitor 400.

Examples of a region of interest (a region of concern) detected in step S103 may include a polyp, a cancer, a colon diverticulum, an inflammation, a treatment scar (a scar of endoscopic mucosal resection (EMR), a scar of endoscopic submucosal dissection (ESD), a clip portion, or the like), a bleeding point, a perforation, angiodysplasia, and the like.

The region-of-interest detecting unit 204F determines whether or not a region of interest has been detected (step S104). If the determination is affirmative (if a region of interest has been detected), the processing proceeds to step S108, where a still image capturing instruction (an acquisition instruction) is provided. If the determination is negative (if a region of interest has not been detected), the processing proceeds to step S106, where it is determined whether or not an instruction to capture a still image has been received from a user (an acquisition instruction reception step). The capturing instruction can be provided by the user by operating the imaging button 144 or the operation unit 208. If the determination in step S106 is negative, the processing returns to step S102, where acquisition and display of a first moving image are repeated (moving image acquisition processing).

### Reception of Still Image Capturing Instruction and Acquisition Instruction

If the determination in step S104 is affirmative (if a region of interest has been detected), the image acquisition control unit 204C provides a still image capturing instruction (an acquisition instruction) (step S108: a still image acquisition instruction step). Also in a case where a user instruction is received in step S106, a still image capturing instruction (an acquisition instruction) is provided in response to the instruction (step S108: a still image acquisition instruction step). The acquisition instruction receiving unit 204B receives the still image capturing instruction (an acquisition instruction) (step S110: an acquisition instruction reception step).

### Capturing of Still Image

In response to receipt of the still image capturing instruction (an acquisition instruction), the image acquiring unit 204A acquires one frame of the first moving image as a first still image under control by the image acquisition control unit 204C (step S112: a still image acquisition step, still image acquisition processing). The frame to be acquired as a first still image can be a frame in which a region of interest has been detected in the above-described processing, and may be another frame (for example, another frame having an imaging time difference smaller than or equal to a threshold value from the frame in which the region of interest has been detected). In addition, the image acquiring unit 204A controls the light source control unit 350 under control by the image acquisition control unit 204C to cause the blue light source 310B to emit light and irradiate the subject with blue light (second observation light) as narrow-band light instead of white light (first observation light), and the imaging optical system 130, the imaging device 134, and so forth capture (acquire) a second still image of the subject (step S114: a still image acquisition step, still image acquisition processing).

### Acquisition Patterns of First Image and Second Image

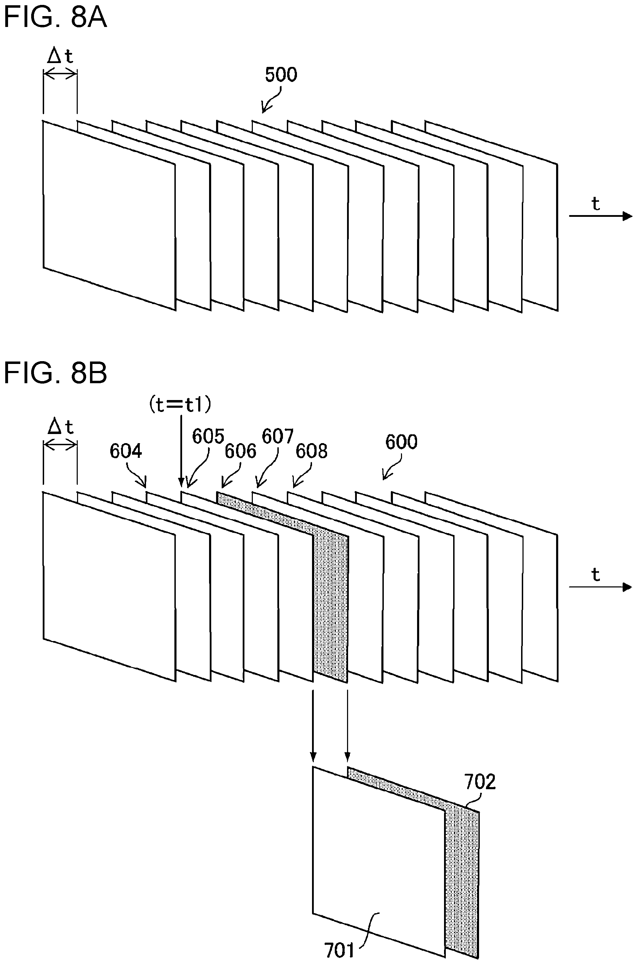

Figs. 8A and 8B are diagrams illustrating examples of acquiring a first image (a first moving image, a first still image), and a second image (a second still image) in the first embodiment. Each of these figures illustrates a state in which images are acquired from the left to the right in the figure along a time axis t. Fig. 8A illustrates a state in which a first moving image 500 (a first image) is continuously captured by using first observation light (white light, normal light) at a designated frame rate (a frame interval: Δt). Fig. 8B illustrates a state in which a still image acquisition instruction is received at the timing t = t1, and in response to the instruction, an image 605 (a first image) in a moving image 600 is acquired as a first still image 701 (step S112) and an image 606 (a second image) is acquired as a second still image 702 (step SI14). Alternatively, a plurality of first and second still images may be captured in response to a still image acquisition instruction.

### Generation of Observation Still Image

The image editing unit 204H performs image processing on the first still image 701 and/or the second still image 702 to generate an observation still image (step S116: an image processing step). The image editing unit 204H is capable of generating a white-light image, a special-light image, and an image of the combination thereof. The image editing unit 204H is also capable of performing image processing, such as color balance adjustment, blood vessel emphasis, feature quantity emphasis, difference emphasis, or combining of images that have undergone these processes, to generate an observation image, a classification (discrimination) image, and the like. For example, the image editing unit 204H is capable of generating a blue-region-emphasized image from a white-light image and a blue-narrow-band-light image. In the blue-region-emphasized image, minute blood vessels in a surface layer of a mucous membrane of an organ, a minute structure of a mucous membrane, or the like can be displayed in an emphasized manner. In addition, the image editing unit 204H is capable of generating a red-region-emphasized image. In the red-region-emphasized image, a small color difference in a red region of the image can be displayed in an emphasized manner. The white-light image is an image suitable for ordinary observation. These observation images enable a user to efficiently perform observation. The image processing to be performed may be determined in accordance with an instruction from the user, or may be determined by the image editing unit 204H without an instruction from the user. The image editing unit 204H is capable of recording the generated observation still image as the observation still image 207E in the recording unit 207.

### Prevention of Decrease in Frame Rate of First Image

In the first embodiment, to prevent degradation of image quality resulting from wavelength separation, only the first observation light or the second observation light is radiated as observation light, and the first observation light and the second observation light are not simultaneously radiated, and thus a first image is not acquired at the radiation timing of the second observation light. For example, in the case of acquiring a first still image and a second still image in the pattern illustrated in Fig. 8B, a first still image is not acquired at the acquisition timing of the image 606 (a second still image). Thus, in the first embodiment, an "alignment first image" ("a first image at the imaging time of a second image, generated by applying an alignment parameter to a first image") is generated and displayed in the manner described below to prevent a substantial decrease in the frame rate of the first image (step S 118: an alignment first image generation step). The details of the processing of generating an alignment first image will be described below.

### Detection of Region of Interest from Second Still Image

The region-of-interest detecting unit 204F detects a region of interest as a photographic subject from a first still image and/or a second still image (step S119: a second region-of-interest detection step). Detection of a region of interest can be performed similarly to the first region-of-interest detection step in step S103. In a case where the frame of the first moving image in which a region of interest is detected in the processing in step S103 has been acquired as a first still image, further detection processing on the first still image can be omitted.

### Classification of Region of Interest

The classifying unit 204E classifies (discriminates) the region of interest (an example of a photographic subject) detected from the second still image in step S119 (step S120: a classification step). Examples of classification may be the type of lesion (hyperplastic polyp, adenoma, intramucosal cancer, invasive cancer, or the like), the range of the lesion, the size of the lesion, the gross appearance of the lesion, diagnosis of the stage of cancer, a current position in a lumen (a pharynx, an esophagus, a stomach, a duodenum, or the like in an upper portion; a cecum, an ascending colon, a transverse colon, a descending colon, a sigmoid colon, a rectum, or the like in a lower portion), and the like. In the classification, a result of machine learning (deep learning) can be used as in the case of detection. The classification of the region of interest may be performed together with detection. In a case where the first observation light is white light and the second observation light is blue narrow-band light, it is preferable that the classifying unit 204E classify the region of interest (a photographic subject) on the basis of at least a second still image of a first still image and a second still image. This is because, in the above-described example, the second still image is captured by using blue narrow-band light whose center wavelength is shorter than that of the first observation light (white light) and is suitable for classifying a minute structure of a lesion or the like. The image to be used to classify the region of interest may be set on the basis of a user operation performed via the operation unit 208 or may be set by the classifying unit 204E without a user operation.

### Display of Still Image

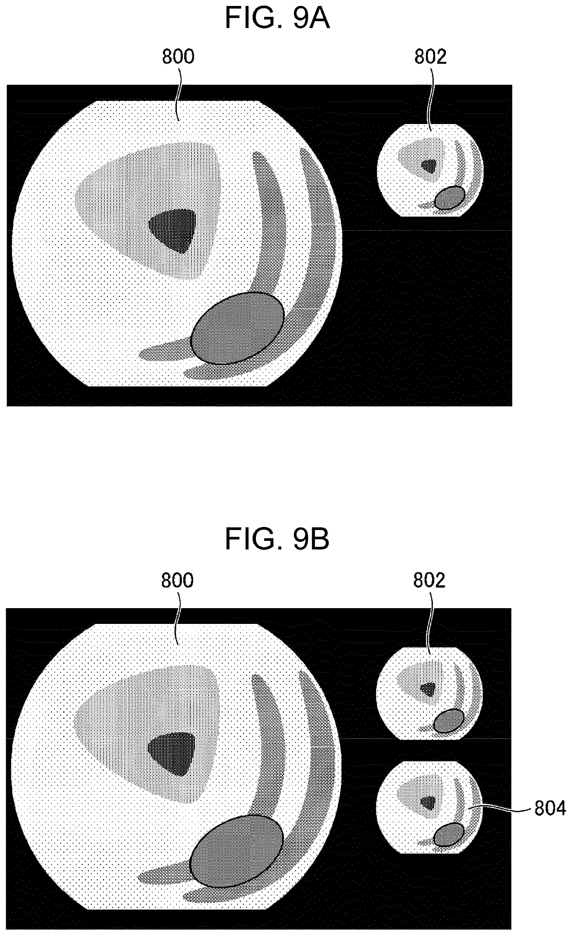

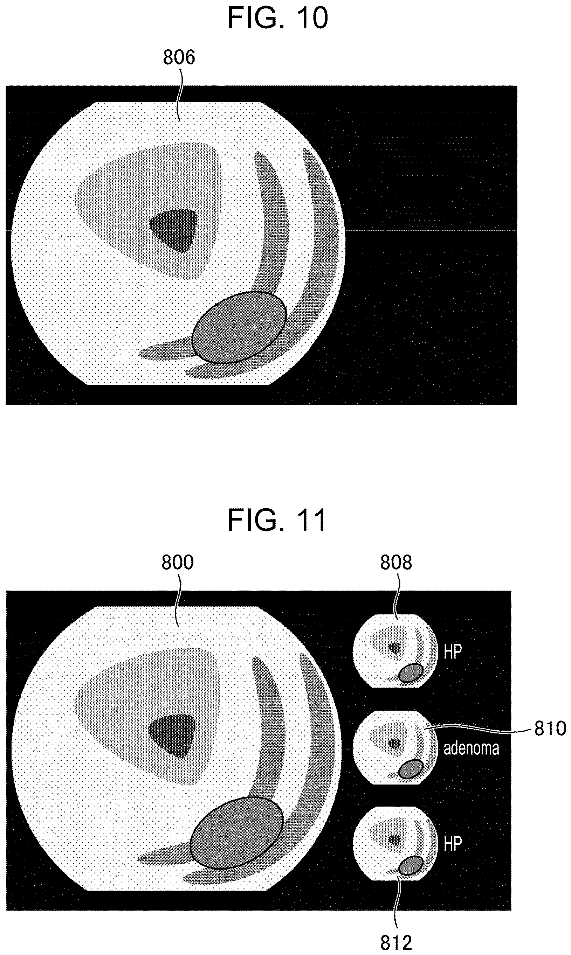

The display control unit 204D causes the monitor 400 (a display apparatus) to display a still image (a first still image, a second still image, an observation still image) (step S122: a still image display step). The still image to be displayed may be the observation still image generated in step S116 as well as the acquired first still image and second still image. These still images can be displayed in various patterns. For example, as illustrated in Fig. 9A, while a moving image 800 is continuously displayed on the monitor 400, a first still image 802 may be displayed in another display region. Alternatively, as illustrated in Fig. 9B, a second still image 804 may be displayed in addition to the first still image 802. The number of still images that are displayed is not limited to one, and a plurality of still images may be displayed. In the case of displaying a plurality of still images, a still image may be added for display every time a still image is acquired, an old still image may be erased when the display region is filled, and then a newly acquired still image may be displayed. Instead of displaying a moving image and a still image side by side as illustrated in Figs. 9A and 9B, only a still image 806 (a first still image and/or a second still image) may be displayed in a frozen manner (the same still image may be continuously displayed) for a certain period, as illustrated in Fig. 10. Display of a still image illustrated as examples in Figs. 9A to 10 enables a user to check the still image, such as an image used for classification (discrimination), during diagnosis (observation), and to provide an instruction to capture an image again if the image has a fault, such as blur, halation, or fogging.

### Display of Classification Result

The display control unit 204D may cause the monitor 400 (a display apparatus) to display information indicating a result of classification together with a still image (step S124: a classification result display step). Fig. 11 is a diagram illustrating a display example of a classification result, in which the moving image 800, still images 808, 810, and 812, and classification results for these still images are shown. In Fig. 11, "HP" represents "helicobacter pylori", and "adenoma" represents "adenoma". Such display of classification results enables a user to simultaneously evaluate the quality of still images and classification (discrimination) results, and to determine which result is reliable in a case where the same lesion has different discrimination results. The classifying unit 204E and the region-of-interest detecting unit 204F may output information indicating a detection result and/or a classification result of a region of interest as sound through the audio processing unit 209 and the speaker 209A.

### Emphasized Display of Region of Interest

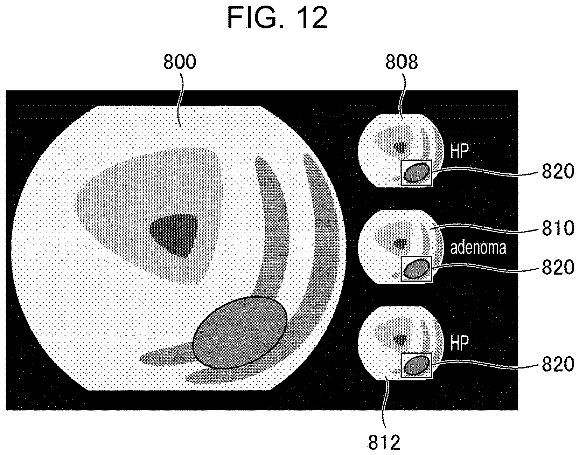

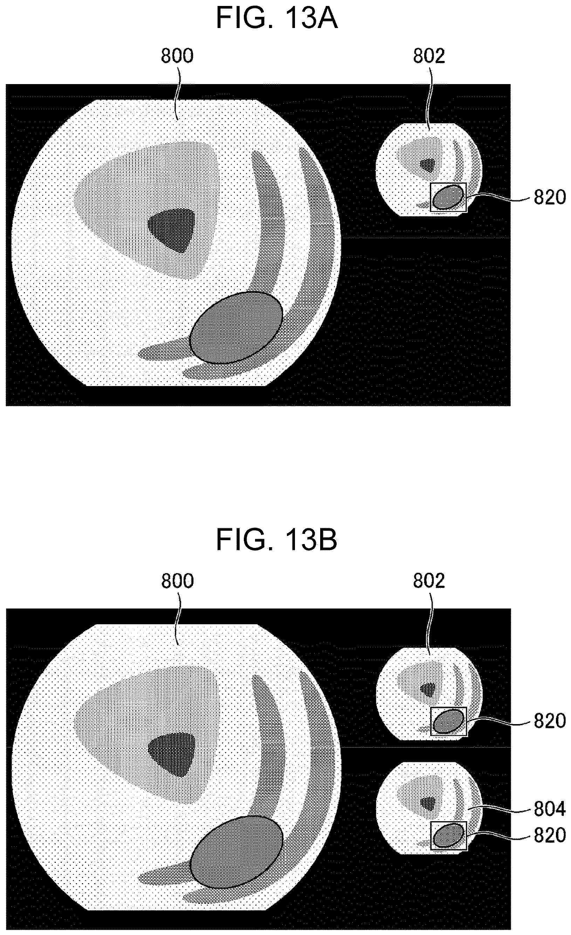

When displaying an image and a classification result, the display control unit 204D, the classifying unit 204E, and the region-of-interest detecting unit 204F are capable of displaying a region of interest in an emphasized manner. Output of information can be performed by, for example, superimposing and displaying characters, numerals, symbols, colors, and the like indicating the position and size of the region of interest on a first still image and/or a second still image by the display control unit 204D, the classifying unit 204E, the region-of-interest detecting unit 204F, and so forth. Fig. 12 illustrates an example of such emphasized display, in which rectangles 820 surrounding the regions of interest as targets to be classified are displayed in addition to the classification result illustrated in Fig. 11. The emphasized display may be performed on the display mode illustrated in Fig. 9A, 9B, or 10 (without displaying a classification result). Figs. 13A and 13B are diagrams illustrating an example in which emphasized display is performed in the modes illustrated in Figs. 9A and 9B. In a case where a first still image and/or a second still image is displayed with a region of interest not being emphasized, the user needs to check the entire image to find a region of interest. When a region of interest is displayed in an emphasized manner in this way, the user is able to easily determine which region is a target of detection or classification of a region of interest. If a region of interest is wrongly detected, it can be easily determined that a region of interest is not included in the first image and/or the second image and that wrong detection has been performed. Emphasizing of a region of interest can be performed through marking with a specific figure, such as a circle, a cross, or an arrow, superimposition processing, change of color tone or gradation, frequency processing, or the like, other than the examples illustrated in Figs. 12 to 13B (display of rectangles), and is not limited to these examples.

### Storage of Classification Result and Image

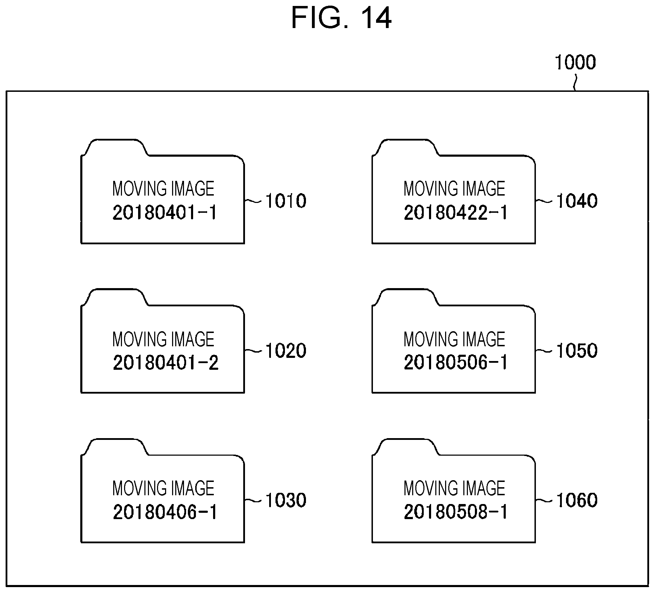

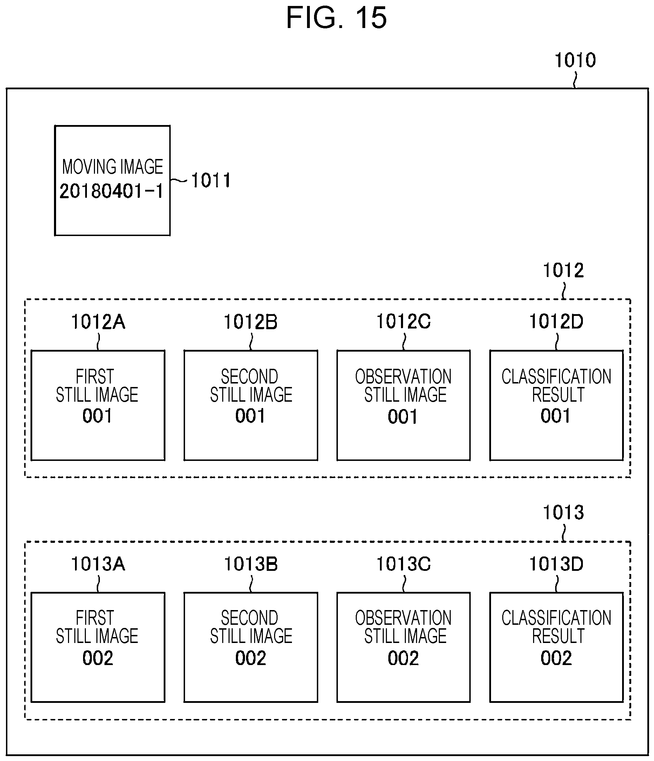

The classification result storing unit 204G stores a result of classification as the region-of-interest classification result 207F in the recording unit 207 in association with the first still image and/or the second still image (step S126: a classification result storage step). The result of classification may be associated with the above-described observation still image. Figs. 14 and 15 are diagrams illustrating examples in which classification results and images are stored in association with each other. Fig. 14 illustrates a state in which subfolders 1010, 1020, 1030, 1040, 1050, and 1060 associated with moving images are stored in a main folder 1000 created in the recording unit 207. Fig. 15 is a diagram illustrating the images and information stored in the subfolder 1010, and illustrates a state in which a moving image 1011 and subfolders 1012 and 1013 associated with the moving image 1011 are stored, and accordingly the moving image 1011 is associated with the subfolders 1012 and 1013. The subfolder 1012 stores a first still image 1012A, a second still image 1012B, an observation still image 1012C, and a classification result 1012D, and these still images are associated with the classification result. Similarly, the subfolder 1013 stores a first still image 1013A, a second still image 1013B, an observation still image 1013C, and a classification result 1013D, and these still images are associated with the classification result. Such storage using folders enables a user to easily grasp the correspondence between images and classification results.

At the time of storing the images and classification results in the above-described manner, an image in which a region of interest, such as a lesion, has been detected (hereinafter referred to as a "lesion image") may be stored (recorded) in association with a test in which a specific lesion (a lesion of low prevalence, a case difficult to be detected, or the like) has been found. For example, in a case where the size of a lesion is small or in a case where the shape of a lesion is flat and hardly has a bump, a lesion image (a still image, a moving image) can be stored as a "lesion difficult to be detected". For example, in a case where pathological biopsy is performed (in this case, it is considered "a lesion subjected to biopsy is difficult to be determined by endoscopic findings") or in a case where a result of pathological biopsy does not match endoscopic findings (for example, biopsy is performed because of endoscopic findings "suspected adenoma" but a pathological result is a hyperplastic polyp), a lesion image can be stored as a "lesion difficult to be diagnosed". Furthermore, in the case of constructing a learner through machine learning by using a lesion image as an input, the lesion mage may be stored in accordance with the usage purpose of the learner. For example, in the case of constructing a learner aimed at detecting (picking out) a lesion in screening, only a test aimed at screening may be stored (manipulation video of endoscopic submucosal dissection (ESD) or the like is of low utility value in machine learning or the like), and in the case of constructing a leaner aimed at determining the stage of cancer (intramucosal cancer, advanced cancer, or the like), only a lesion image of a test aimed at treatment, such as ESD or endoscopic mucosal resection (EMR), may be stored.

After the classification result and the image have been stored, the image processing unit 204 (the image acquisition control unit 204C) determines whether or not to finish the processing of the image processing method (step S128: a termination determination step). In the case of continuing the processing (NO in step S128), the still image acquisition processing (acquisition and display or the like of first and second still images) is finished, and the processing returns to step S102, where the moving image acquisition processing is restarted.

### Processing for Alignment First Image

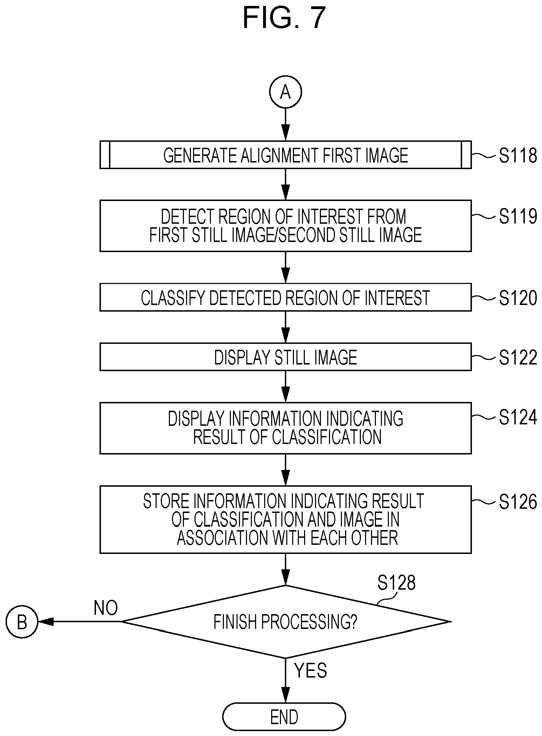

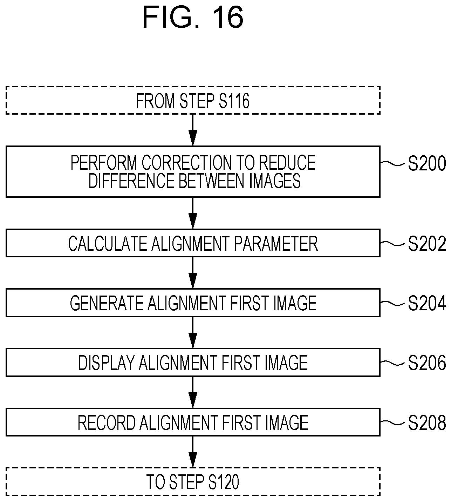

Hereinafter, the details of the processing for an alignment first image in step S118 in Fig. 7 will be described with reference to the flowchart in Fig. 16.

### Image Used to Generate Alignment First Image

To generate an alignment first image, a first still image (the image 605) acquired before a first still image absence timing (an imaging timing of the image 606, which is a second still image), for example, the image 605 (the first still image 701) and the image 606 (the second still image 702) in Fig. 8B, can be used. Specifically, "a first still image captured at an imaging time that is before an imaging time of a second still image and that has a temporal difference smaller than or equal to a first threshold value from the imaging time of the second still image" can be used. Accordingly, an alignment first image can be generated with a small change in the tint and structure of a photographic subject between frames. The threshold value for the imaging time (the first threshold value) can be determined in accordance with alignment accuracy, an allowable time for delay in generation and display of an image, and so forth. Hereinafter, a description will be given of the case of generating an alignment first image by using the image 605 as a first still image (the first still image 701) and the image 606 as a second still image (the second still image 702).

In the generation of the alignment first image, other than the above-described pattern, for example, a plurality of first still images whose imaging times are different (in Fig. 8B, for example, images 604 and 605) may be used, or first still images acquired after a first still image absence timing (in Fig. 8B, for example, images 607 and 608) may be used. For example, in the case of a system in which a delay occurs from acquisition to display of an image, it is possible to achieve alignment by using first images captured after the imaging time of a second image (for example, the images 607 and 608), and it is also possible to achieve highly accurate alignment by performing alignment by using first images captured before and after the imaging time of a second image (for example, the images 605 and 607).

### Correction Before Alignment (Preprocessing)

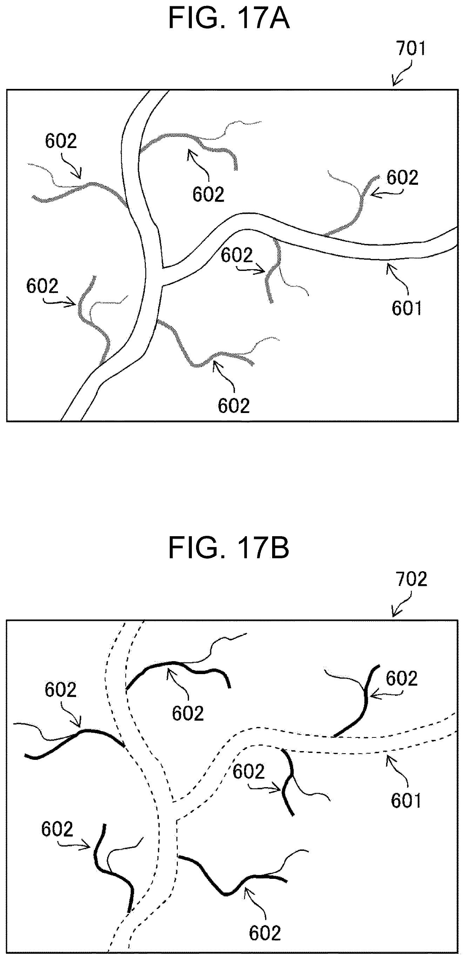

The first still image and the second still image are different in the wavelength of observation light as well as in imaging timing. Accordingly, in the first still image 701 using white light as observation light, thick blood vessels 601 are clearly seen but thin blood vessels 602 are not clearly seen as illustrated in Fig. 17A, for example. In contrast, in the second still image 702 using blue narrow-band light as observation light, the thick blood vessels 601 are not clearly seen but the thin blood vessels 602 are clearly seen as illustrated in Fig. 17B, for example, compared with the first still image 701. Thus, in the first embodiment, the image processing unit 204 (the parameter calculating unit 2041) performs correction (preprocessing) for reducing the difference between the first still image and the second still image caused by the difference between the first observation light and the second observation light (step S200: an image correction step).

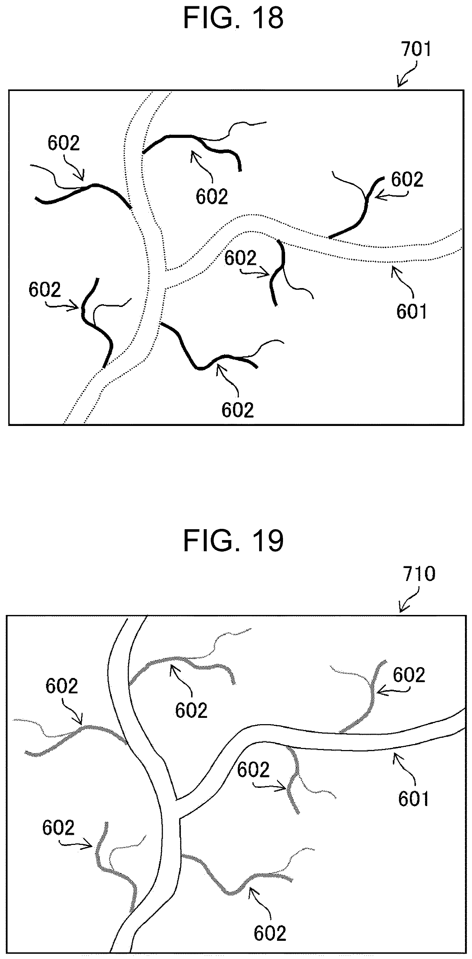

Specifically, the parameter calculating unit 2041 extracts a wavelength component common to the first observation light and the second observation light in an image signal of the first still image and an image signal of the second still image, weights at least one of the image signal of the first still image or the image signal of the second still image with the extracted wavelength component, and generates an image in which the signal intensity of the common wavelength component is higher than the signal intensity of components other than the common wavelength component. In the first embodiment, the first observation light is white light and the second observation light is blue light, and thus the parameter calculating unit 2041 increases the weight of a blue light component which is a wavelength common to the image signal of the first still image and the image signal of the second still image. Fig. 18 illustrates an example of a state in which a blue light component is weighted in the first still image 701, where the thin blood vessels 602 are relatively emphasized.

In the first embodiment, the alignment accuracy can be increased by such correction (preprocessing), and an image (an alignment first image) with a small change in the tint and structure of a photographic subject between frames can be acquired. Alternatively, an alignment first image may be generated by using only a common wavelength component instead of weighting the common wavelength component (a blue light component) as described above.

### Calculation of Parameter and Alignment

The parameter calculating unit 2041 calculates a parameter for achieving matching between the corrected (preprocessed) first still image 701 and the second still image 702 by alignment (step S202: a parameter calculation step). The parameter to be calculated is a parameter about at least one of relative movement, rotation, or deformation, and "deformation" may include enlargement or reduction. The image generating unit 204J applies the generated parameter to the corrected first still image 701 to generate an alignment first image (step S204: an image generation step). In steps S202 and S204, the parameter calculating unit 2041 calculates a parameter for performing projective transformation between the first still image and the second still image, and the image generating unit 204J performs projective transformation based on the calculated parameter on the first still image, and thereby being capable of generating an alignment first image. An example of the alignment first image (an image 710) is illustrated in Fig. 19. As described above, although the second still image is used to calculate the parameter, the alignment first image is generated by moving or deforming the first still image, and thus the tint of the alignment first image is not changed by an influence of pixel values of the second still image.

### Output about Alignment First Image

The display control unit 204D and the image generating unit 204J cause the monitor 400 (a display apparatus) to display the alignment first image (step S206: a display control step). In addition, the display control unit 204D and the image generating unit 204J record the generated alignment first image as the "alignment first image 207D" in the recording unit 207 (step S208: an alignment first image recording step). Display and recording of the alignment first image can be sequentially performed after display and recording of individual frames of the first moving image. Such sequential display may be performed in real time during a test of a photographic subject, or may be performed when a user views the first moving image and the alignment first image (the first moving image 207A and the alignment first image 207D) recorded in the recording unit 207 later. In a case where the alignment first image is generated by performing the above-described correction (weighting of a blue light component) on the first image, the image generating unit 204J may change the balance of wavelength components to the original balance so as be the same as white light when the alignment first image is output (displayed or the like). Accordingly, it is possible to prevent that an image of different wavelength balance is displayed on the monitor 400 and the user feels unnatural.

In this way, in the first embodiment, it is possible to display an alignment first image (step S206) even at the timing when a first image is not acquired, in addition to display a normal first moving image (step S102). Accordingly, a substantial decrease in the frame rate of the first moving image can be prevented, and the user is able to continue observation by using a normal-light image (a first image) captured by using normal light (white light).

In the flowcharts in Figs. 6 and 7, a description has been given of a case where an alignment first image is displayed at a timing when a first image is not acquired. Alternatively, the display control unit 204D may continue displaying a first image instead of displaying an alignment first image. In this case, it is preferable that, at the timing of acquiring a second image, the display control unit 204D cause the monitor 400 (a display apparatus) to display a first image captured at an imaging time that has a temporal difference smaller than or equal to a second threshold value from an imaging time of a second image. For example, when the second threshold value is 2 ×Δt (Δt is a frame interval of a moving image), in the example illustrated in Fig. 8B, display of the images 605 and 607 (the temporal difference is Δt) or the images 604 and 608 (the temporal difference is 2 × Δt) can be continued at the timing to acquire the image 606, which is a second image.

As described above, in the endoscope system 10 according to the first embodiment, a user is able to, while continuing observation with a first image, acquire a still image by using first or second observation light as necessary (at the timing when acquisition of a still image is necessary, for example, when a user instruction is provided or when a region of interest is detected), and to classify a photographic subject while performing observation. In addition, generation and display of an alignment first image make it possible to acquire an image with a small change in the tint and structure of a photographic subject between frames while preventing a substantial decrease in the frame rate of display of an image (a first image), and accordingly an accurate structure of the photographic subject can be observed. In this way, the endoscope system 10 is capable of acquiring images by using a plurality of types of observation light as necessary while suppressing an influence on observation performed by a user.

### Other Configurations of Light Source and Effect of Applying the Present Invention

A description will be given of examples of another configuration of a light source in the endoscope system according to the present invention and an effect of applying the image processing apparatus of the present invention in that case.

### Example 1

As illustrated in Fig. 20, a light source apparatus 320 (a light source apparatus) includes a white-light laser light source 312 (a white-light laser light source) that radiates white-light laser as excitation light, a fluorescent body 314 (a fluorescent body) that emits white light as first observation light when irradiated with white-light laser, and a narrow-band-light laser light source 316 (a narrow-band-light laser light source) that radiates narrow-band light as second observation light (for example, blue narrow-band light, or green narrow-band light or red narrow-band light). The light source apparatus 320 is controlled by the light source control unit 350. In Fig. 20, illustration of the components of the endoscope system 10 is omitted, except for the light source apparatus 320 and the light source control unit 350.

In the case of using the white-light laser light source 312 to acquire white light as first observation light, if the number of times of acquisition of a second image is large, repetition of radiation and non-radiation of first observation light increases. Thus, repetition of excitation and non-excitation of the white-light laser light source 312 increases, which may hasten degradation of the light source. However, since the endoscope system 10 includes the image processing apparatus according to the present invention, an advantageous effect of including the image processing apparatus is acquired. That is, a second image is acquired only when an instruction to acquire a still image is provided (when a region of interest is detected, when a user instruction is provided), and a second image is not acquired when an acquisition instruction is not provided (for example, when a region of interest is not detected and classification is not necessary). Thus, it is possible to prevent that increased repetition of radiation and non-radiation of first observation light unnecessarily hastens degradation of the light source.

### Example 2

As illustrated in Fig. 21, a light source apparatus 322 (a light source apparatus) includes a white light source 318 (a white light source) that emits white light, a rotary filter 360 (a white-light filter, a narrow-band-light filter) in which a white-light region that allows white light to pass therethrough and a narrow-band-light region that allows narrow-band light to pass therethrough are formed, and a rotary filter control unit 363 (a first filter switching control unit) that controls rotation of the rotary filter 360 to insert the white-light region or the narrow-band-light region to the optical path of white light. The white light source 318 and the rotary filter control unit 363 are controlled by the light source control unit 350. In Fig. 21, illustration of the components of the endoscope system 10 is omitted, except for the light source apparatus 322 and the light source control unit 350.

In the case of generating a plurality of types of observation light (for example, white light as first observation light and narrow-band light as second observation light) by controlling the rotation of the rotary filter 360, lack of synchronization between the rotation of the rotary filter 360 and the read-out timing of the image sensor (the imaging device 134) may cause an imbalance in the color of a first image and/or a second image. However, since the endoscope system 10 includes the image processing apparatus according to the present invention, an advantageous effect of including the image processing apparatus is acquired. That is, a second image is acquired only when an instruction to acquire a still image is provided (when a region of interest is detected, when a user instruction is provided), and a second image is not acquired when an acquisition instruction is not provided (for example, when a region of interest is not detected and classification is not necessary). Thus, it is possible to reduce the possibility that the number of times of switching of the light source or the filter increases and the color balance of a first image and/or a second image is lost.

In example 2, the white light source 318 may use a white light source that emits wide-band light, or may generate white light by causing light sources that emit red light, blue light, and green light to simultaneously radiate light. In addition, the rotary filter 360 and the rotary filter control unit 363 may be provided in the light source 310 illustrated in Fig. 2.

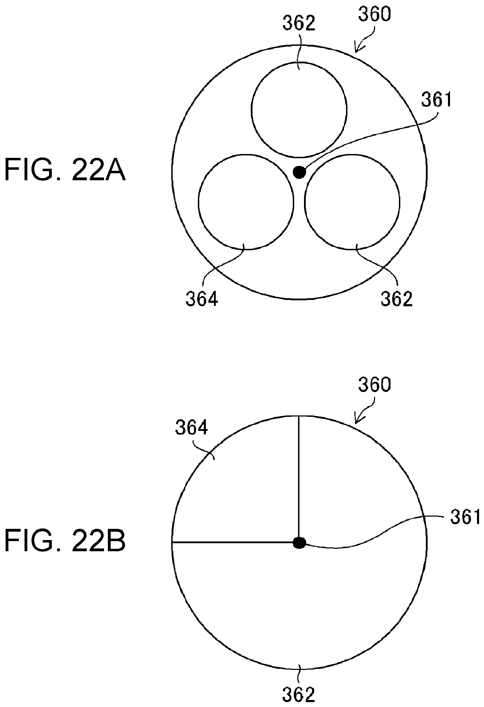

Figs. 22A and 22B are diagrams illustrating examples of the rotary filter 360. In the example illustrated in Fig. 22A, two circular white-light regions 362 (white-light filters) that allow white light to pass therethrough and one circular narrow-band-light region 364 (a narrow-band-light filter) that allows narrow-band light to pass therethrough are formed in the rotary filter 360. By rotating the rotary filter 360 around a rotational axis 361 under control by the rotary filter control unit 363 (a first filter switching control unit), the white-light region 362 or the narrow-band-light region 364 is inserted to the optical path of white light, and accordingly a subject is irradiated with white light or narrow-band light. The narrow-band-light region 364 can be a region that allows any narrow-band light, such as red narrow-band light, blue narrow-band light, green narrow-band light, or purple narrow-band light, to pass therethrough. The number, shapes, and arrangement of white-light regions 362 and narrow-band-light regions 364 are not limited to the example illustrated in Fig. 22A and may be changed in accordance with the radiation ratio of white light and narrow-band light.

The shapes of the white-light region and the narrow-band-light region are not limited to circular as illustrated in Fig. 22A and may be a fan-shape as illustrated in Fig. 22B. Fig. 22B illustrates an example in which 3/4 of the rotary filter 360 is used as the white-light region 362 and 1/4 of the rotary filter 360 is used as the narrow-band-light region 364. The area of the fan-shape can be changed in accordance with the radiation ratio of white light and narrow-band light. In the examples in Figs. 22A and 22B, a plurality of narrow-band-light regions corresponding to different types of narrow-band light may be provided in the rotary filter 360.

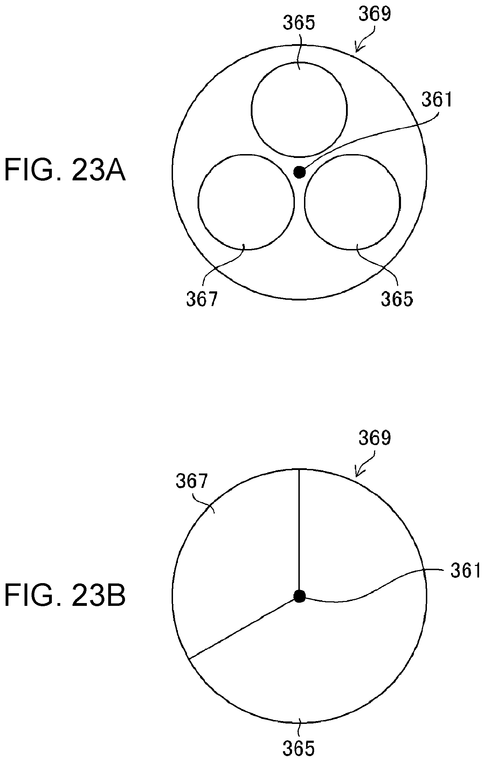

Figs. 23A and 23B are diagrams illustrating other examples of the rotary filter. As a white light source for the rotary filters illustrated in Figs. 23A and 23B, the white light source 318 can be used as in the light source apparatus 322 illustrated in Fig. 21. A rotary filter 369 illustrated in Fig. 23A is not provided with a white-light region that allows white light to pass therethrough, unlike the rotary filter 360 illustrated inFigs. 22A and 22B, but is provided with two circular first-narrow-band-light regions 365 (first-narrow-band-light filters) that allow a component of first narrow-band light in white light to pass therethrough and one circular second-narrow-band-light region 367 (a second-narrow-band-light filter) that allows a component of second narrow-band light in white light to pass therethrough. By rotating the rotary filter 369 around the rotational axis 361 under control by the rotary filter control unit 363 (see Fig. 21; a second filter switching control unit), the first-narrow-band-light region 365 (a first-narrow-band-light filter) or the second-narrow-band-light region 367 (a second-narrow-band-light filter) is inserted to the optical path of white light emitted by the white light source 318, and accordingly a subject can be irradiated with first narrow-band light or second narrow-band light.

The shapes of the first-narrow-band-light regions 365 and the second-narrow-band-light region 367 are not limited to circular as illustrated in Fig. 23A and may be a fan-shape as illustrated in Fig. 23B. Fig. 23B illustrates an example in which 2/3 of the rotary filter 369 is used as the first-narrow-band-light region 365 and 1/3 of the rotary filter 369 is used as the second-narrow-band-light region 367. The area of the fan-shape can be changed in accordance with the radiation ratio of first narrow-band light and second narrow-band light. In the examples in Figs. 23A and 23B, three or more narrow-band-light regions corresponding to different types of narrow-band light may be provided in the rotary filter 369.

In the case of generating a plurality of types of observation light (first narrow-band light and second narrow-band light) by switching the filter by the rotary filter control unit 363, lack of synchronization between switching of the filter and the read-out timing of the image sensor (the imaging device 134) may cause an imbalance in the color of a first image and/or a second image. However, since the endoscope system 10 includes the image processing apparatus according to the present invention, an advantageous effect of including the image processing apparatus is acquired. That is, a second image is not acquired when an instruction to acquire a second image is not provided (for example, when a region of interest is not detected and classification is not necessary). Thus, it is possible to reduce the possibility that the number of times of switching of the light source or the filter increases and the color balance of a first image and/or a second image is lost.

### Appendices

In addition to the individual aspects of the above-described embodiment, the configurations described below are included in the scope of the present invention.

### Appendix 1

A medical image processing apparatus wherein
a medical image analysis processing unit detects a region of interest on the basis of a feature quantity of pixels of a medical image, the region of interest being a region to be focused on, and
a medical image analysis result acquiring unit acquires an analysis result of the medical image analysis processing unit.

### Appendix 2

A medical image processing apparatus wherein
a medical image analysis processing unit detects presence or absence of a target to be focused on on the basis of a feature quantity of pixels of a medical image, and
a medical image analysis result acquiring unit acquires an analysis result of the medical image analysis processing unit.

### Appendix 3

The medical image processing apparatus wherein
the medical image analysis result acquiring unit acquires the analysis result of the medical image from a recording device in which the analysis result is recorded, and
the analysis result is either or both of the region of interest which is a region to be focused on included in the medical image and the presence or absence of the target to be focused on.

### Appendix 4

The medical image processing apparatus wherein the medical image is a normal-light image acquired by radiating light in a white range or light in a plurality of wavelength ranges as the light in the white range.

### Appendix 5

The medical image processing apparatus wherein
the medical image is an image acquired by radiating light in a specific wavelength range, and
the specific wavelength range is a range narrower than a white wavelength range.

### Appendix 6

The medical image processing apparatus wherein the specific wavelength range is a blue or green range in a visible range.

### Appendix 7

The medical image processing apparatus wherein the specific wavelength range includes a wavelength range of 390 nm or more and 450 nm or less or a wavelength range of 530 nm or more and 550 nm or less, and the light in the specific wavelength range has a peak wavelength in the wavelength range of 390 nm or more and 450 nm or less or the wavelength range of 530 nm or more and 550 nm or less.

### Appendix 8

The medical image processing apparatus wherein the specific wavelength range is a red range in a visible range.

### Appendix 9

The medical image processing apparatus wherein the specific wavelength range includes a wavelength range of 585 nm or more and 615 nm or less or a wavelength range of 610 nm or more and 730 nm or less, and the light in the specific wavelength range has a peak wavelength in the wavelength range of 585 nm or more and 615 nm or less or the wavelength range of 610 nm or more and 730 nm or less.

### Appendix 10

The medical image processing apparatus wherein the specific wavelength range includes a wavelength range in which a light absorption coefficient is different between oxyhemoglobin and deoxyhemoglobin, and the light in the specific wavelength range has a peak wavelength in the wavelength range in which the light absorption coefficient is different between oxyhemoglobin and deoxyhemoglobin.

### Appendix 11

The medical image processing apparatus wherein the specific wavelength range includes a wavelength range of 400 ± 10 nm, a wavelength range of 440 ± 10 nm, a wavelength range of 470 ± 10 nm, or a wavelength range of 600 nm or more and 750 nm or less, and the light in the specific wavelength range has a peak wavelength in the wavelength range of 400 ± 10 nm, the wavelength range of 440 ± 10 nm, the wavelength range of 470 ± 10 nm, or the wavelength range of 600 nm or more and 750 nm or less.

### Appendix 12

The medical image processing apparatus wherein
the medical image is an inside-of-living-body image depicting an inside of a living body, and
the inside-of-living-body image has information about fluorescence emitted by a fluorescent substance in the living body.

### Appendix 13

The medical image processing apparatus wherein the fluorescence is acquired by irradiating the inside of the living body with excitation light whose peak is 390nm or more and 470 nm or less.

### Appendix 14

The medical image processing apparatus wherein
the medical image is an inside-of-living-body image depicting an inside of a living body, and
the specific wavelength range is a wavelength range of infrared light.

### Appendix 15

The medical image processing apparatus wherein the specific wavelength range includes a wavelength range of 790 nm or more and 820 nm or less or a wavelength range of 905 nm or more and 970 nm or less, and the light in the specific wavelength range has a peak wavelength in the wavelength range of 790 nm or more and 820 nm or less or the wavelength range of 905 nm or more and 970 nm or less.

### Appendix 16

The medical image processing apparatus wherein
a medical image acquiring unit includes a special-light image acquiring unit that acquires a special-light image having information about the specific wavelength range on the basis of a normal-light image that is acquired by radiating light in a white range or light in a plurality of wavelength ranges as the light in the white range, and
the medical image is the special-light image.

### Appendix 17

The medical image processing apparatus wherein a signal in the specific wavelength range is acquired through computation based on color information of RGB or CMY included in the normal-light image.

### Appendix 18

The medical image processing apparatus including
a feature quantity image generating unit that generates a feature quantity image through computation based on at least one of a normal-light image or a special-light image, the normal-light image being acquired by radiating light in a white range or light in a plurality of wavelength ranges as the light in the white range, the special-light image being acquired by radiating light in a specific wavelength range, wherein
the medical image is the feature quantity image.

### Appendix 19

An endoscope apparatus including:
the medical image processing apparatus according to any one of appendices 1 to 18; and
an endoscope that acquires an image by radiating at least any one of light in a white wavelength range or light in a specific wavelength range.

### Appendix 20

A diagnosis assistance apparatus including the medical image processing apparatus according to any one of appendices 1 to 18.

### Appendix 21

A medical work assistance apparatus including the medical image processing apparatus according to any one of appendices 1 to 18.

The embodiment of the present invention and other aspects have been described above. The present invention is not limited to the above-described aspects and various modifications can be made without deviating from the spirit of the present invention. Reference Signs List

- 10: endoscope system
- 100: endoscope main body
- 102: handheld operation section
- 104: insertion section
- 106: universal cable
- 108: light guide connector
- 112: soft part
- 114: bending part
- 116: tip rigid part
- 116A: distal-end-side surface
- 123: illumination unit
- 123A: illumination lens
- 123B: illumination lens
- 126: forceps port
- 130: imaging optical system
- 132: imaging lens
- 134: imaging device
- 136: driving circuit
- 138: AFE
- 141: air/water supply button
- 142: suction button
- 143: function button
- 144: imaging button
- 170: light guide
- 200: processor
- 202: image input controller
- 204: image processing unit
- 204A: image acquiring unit
- 204B: acquisition instruction receiving unit
- 204C: image acquisition control unit
- 204D: display control unit
- 204E: classifying unit
- 204F: region-of-interest detecting unit
- 204G: classification result storing unit
- 204H: image editing unit
- 2041: parameter calculating unit
- 204J: image generating unit
- 205: communication control unit
- 206: video output unit
- 207: recording unit
- 207A: first moving image
- 207B: first still image
- 207C: second still image
- 207D: alignment first image
- 207E: observation still image
- 207F: region-of-interest classification result
- 208: operation unit
- 209: audio processing unit
- 209A: speaker
- 210: CPU
- 211: ROM
- 212: RAM
- 300: light source apparatus
- 310: light source
- 310B: blue light source
- 310G: green light source
- 310R: red light source
- 312: white-light laser light source
- 314: fluorescent body
- 316: narrow-band-light laser light source
- 318: white light source
- 320: light source apparatus
- 322: light source apparatus
- 330: diaphragm
- 340: condenser lens
- 350: light source control unit
- 360: rotary filter
- 361: rotational axis
- 362: white-light region
- 363: rotary filter control unit
- 364: narrow-band-light region
- 365: first-narrow-band-light region
- 367: second-narrow-band-light region
- 369: rotary filter
- 400: monitor
- 500: first moving image
- 600: moving image
- 601: blood vessel
- 602: blood vessel
- 604: image
- 605: image
- 606: image
- 607: image
- 608: image
- 701: first still image
- 702: second still image
- 710: image
- 800: moving image
- 802: first still image
- 804: second still image
- 806: still image
- 808: still image
- 810: still image
- 812: still image
- 820: rectangle
- 1000: main folder
- 1010: subfolder
- 1011: moving image
- 1012: subfolder
- 1012A: first still image
- 1012B: second still image
- 1012C: observation still image
- 1012D: classification result
- 1013: subfolder
- 1013A: first still image
- 1013B: second still image
- 1013C: observation still image
- 1013D: classification result
- 1020: subfolder
- 1030: subfolder
- 1040: subfolder
- 1050: subfolder
- 1060: subfolder
- S 100 to S208: individual steps of image processing method
- t: time axis

## Claims

1. An image processing apparatus comprising:
an image acquiring unit that acquires a first image captured by using first observation light and a second image captured by using second observation light different from the first observation light;
an acquisition instruction receiving unit that receives an acquisition instruction to acquire a still image;
an image acquisition control unit that controls acquisition of the first image and the second image by the image acquiring unit;
a display control unit that causes a display apparatus to display at least the first image; and
a classifying unit that performs classification of at least a photographic subject that is seen in the second image, wherein
the image acquisition control unit
causes the image acquiring unit to perform moving image acquisition processing of continuously acquiring the first image as a moving image until the acquisition instruction receiving unit receives the acquisition instruction,
causes the image acquiring unit to perform still image acquisition processing of acquiring the first image and the second image as still images in response to receipt of the acquisition instruction, and
causes the image acquiring unit to perform the moving image acquisition processing after the still image acquisition processing has finished.

2. The image processing apparatus according to claim 1, further comprising
a region-of-interest detecting unit that detects a region of interest from the first image acquired as the moving image, wherein
in a case where the region of interest has been detected, the image acquisition control unit instructs the acquisition instruction receiving unit to acquire the first image and the second image as the still images.

3. The image processing apparatus according to claim 2, wherein
the region-of-interest detecting unit detects the region of interest as the photographic subject from the first image and/or the second image as the still image, and
the display control unit causes the display apparatus to display the first image and/or the second image as the still image such that the detected region of interest is emphasized.

4. The image processing apparatus according to any one of claims 1 to 3, further comprising
a classification result storing unit that stores a result of the classification in association with the first image and/or the second image.

5. The image processing apparatus according to any one of claims 1 to 4, wherein the display control unit causes the display apparatus to display information indicating a result of the classification.

6. The image processing apparatus according to claim 1 or 2, wherein the display control unit causes the display apparatus to display the first image and/or the second image as the still image.

7. The image processing apparatus according to any one of claims 1 to 3, further comprising
an image editing unit that performs image processing on the first image and/or the second image as the still image, wherein
the display control unit causes the display apparatus to display an image acquired through the image processing.

8. The image processing apparatus according to any one of claims 1 to 7, further comprising:
a parameter calculating unit that calculates a parameter for aligning the first image and the second image; and
an image generating unit that generates an alignment first image by applying the parameter to the first image, wherein
the display control unit causes the display apparatus to display the alignment first image at a timing when the second image is acquired.

9. The image processing apparatus according to claim 8, wherein the parameter calculating unit calculates the parameter for aligning the second image and the first image, the first image being captured at an imaging time that has a temporal difference smaller than or equal to a first threshold value from an imaging time of the second image.

10. The image processing apparatus according to claim 8, wherein the parameter calculating unit
extracts a common wavelength component in an image signal of the first image and an image signal of the second image, the common wavelength component being common to a wavelength of the first observation light and a wavelength of the second observation light,
performs at least any one of processing of weighting an image signal component of the first image of the common wavelength component to generate an image signal in which the image signal component of the first image of the common wavelength component is stronger than an image signal component of the first image of a component other than the common wavelength component, or processing of weighting an image signal component of the second image of the common wavelength component to generate an image signal in which the image signal component of the second image of the common wavelength component is stronger than an image signal component of the second image of a component other than the common wavelength component, and
calculates a parameter for aligning the first image and the second image.

11. The image processing apparatus according to claim 8, wherein the parameter calculating unit
extracts a common wavelength component in an image signal of the first image and an image signal of the second image, the common wavelength component being common to a wavelength of the first observation light and a wavelength of the second observation light,
generates an image signal component of the first image of the common wavelength component and an image signal component of the second image of the common wavelength component, and
calculates a parameter for aligning the first image and the second image.

12. The image processing apparatus according to any one of claims 1 to 11, wherein at a timing when the second image is acquired, the display control unit causes the display apparatus to display the first image captured at an imaging time that has a temporal difference smaller than or equal to a second threshold value from an imaging time of the second image.

13. The image processing apparatus according to any one of claims 1 to 12, wherein the image acquiring unit acquires, as the second image, an image captured by using the second observation light, the second observation light being light whose center wavelength is shorter than a center wavelength of the first observation light.

14. The image processing apparatus according to any one of claims 1 to 13, wherein the acquisition instruction receiving unit receives, as the acquisition instruction, an acquisition instruction to acquire a still image from a user.

15. An endoscope system comprising:
the image processing apparatus according to any one of claims 1 to 14;
the display apparatus;
an endoscope that has an insertion section and a handheld operation section, the insertion section being to be inserted into a subject and having a tip rigid part, a bending part connected to a base end side of the tip rigid part, and a soft part connected to a base end side of the bending part, the handheld operation section being connected to a base end side of the insertion section;
a light source apparatus that irradiates the subject with the first observation light or the second observation light; and
an imaging unit that has an imaging lens which forms an optical image of the subject and an imaging device on which the optical image is formed by the imaging lens, wherein
the imaging lens is provided at the tip rigid part.

16. The endoscope system according to claim 15, wherein the light source apparatus irradiates the subject with the first observation light, the first observation light being white light including light in a red wavelength range, a blue wavelength range, and a green wavelength range, and irradiates the subject with the second observation light, the second observation light being narrow-band light corresponding to any one of the red wavelength range, the blue wavelength range, and the green wavelength range.

17. The endoscope system according to claim 16, wherein the light source apparatus comprises a white-light laser light source that radiates white-light laser as excitation light; a fluorescent body that emits the white light as the first observation light when irradiated with the white-light laser; and a narrow-band-light laser light source that radiates the narrow-band light as the second observation light.

18. The endoscope system according to claim 16, wherein the light source apparatus comprises a white light source that emits the white light; a white-light filter that allows the white light to pass therethrough; a narrow-band-light filter that allows a component of the narrow-band light in the white light to pass therethrough; and a first filter switching control unit that inserts the white-light filter or the narrow-band-light filter to an optical path of the white light emitted by the white light source.

19. The endoscope system according to claim 15, wherein the light source apparatus irradiates the subject with the first observation light, the first observation light being first narrow-band light that corresponds to any one of a red wavelength range, a blue wavelength range, and a green wavelength range, and irradiates the subject with the second observation light, the second observation light being second narrow-band light that corresponds to any one of the red wavelength range, the blue wavelength range, and the green wavelength range and that has a wavelength range different from a wavelength range of the first narrow-band light.

20. The endoscope system according to claim 19, wherein the light source apparatus comprises a white light source that emits white light including light in the red wavelength range, the blue wavelength range, and the green wavelength range; a first-narrow-band-light filter that allows a component of the first narrow-band light in the white light to pass therethrough; a second-narrow-band-light filter that allows a component of the second narrow-band light in the white light to pass therethrough; and a second filter switching control unit that inserts the first-narrow-band-light filter or the second-narrow-band-light filter to an optical path of the white light emitted by the white light source.

21. An image processing method for an image processing apparatus comprising an image acquiring unit that acquires a first image captured by using first observation light and a second image captured by using second observation light different from the first observation light, the image processing method comprising:
an acquisition instruction reception step of receiving an acquisition instruction to acquire a still image;
an image acquisition control step of controlling acquisition of the first image and the second image by the image acquiring unit;
a display control step of causing a display apparatus to display at least the first image; and
a classification step of performing classification of at least a photographic subject that is seen in the second image, wherein
the image acquisition control step
causes the image acquiring unit to perform moving image acquisition processing of continuously acquiring the first image as a moving image until the acquisition instruction is received in the acquisition instruction reception step,
causes the image acquiring unit to perform still image acquisition processing of acquiring the first image and the second image as still images in response to receipt of the acquisition instruction, and
causes the image acquiring unit to perform the moving image acquisition processing after the still image acquisition processing has finished.
